(19) Europäisches Patentamt
European Patent Office
Office européen des brevets

(11) Veröffentlichungsnummer: **0 175 250**
**B1**

(12) # EUROPÄISCHE PATENTSCHRIFT

(45) Veröffentlichungstag der Patentschrift: **09.05.90**

(51) Int. Cl.⁵: **C 12 Q 1/28,** C 07 F 9/38, C 07 F 9/30

(21) Anmeldenummer: **85111352.2**

(22) Anmeldetag: **09.09.85**

(54) **Mittel und Verfahren zum Nachweis von Redox-Systemen.**

(30) Priorität: **15.09.84 DE 3433946**

(43) Veröffentlichungstag der Anmeldung:
**26.03.86 Patentblatt 86/13**

(45) Bekanntmachung des Hinweises auf die Patenterteilung:
**09.05.90 Patentblatt 90/19**

(84) Benannte Vertragsstaaten:
**AT BE CH DE FR GB IT LI LU NL SE**

(56) Entgegenhaltungen:
**EP-A-0 007 787**
**EP-A-0 068 356**
**DE-A-3 037 342**

**CHEMICAL ABSTRACTS, Band 96, Nr. 4, 25. Januar 1982, Seite 182, Zusammenfassung Nr. 22914k, Columbus, Ohio, US; & SU-A-839 574 (ALL-UNION SCIENTIFIC-RESEARCH AND PLANNING INSTITUTE FOR MECHANICAL PROCESSING OF MINERALS) 23-06-1981**

(73) Patentinhaber: **BOEHRINGER MANNHEIM GMBH**
**Sandhofer Strasse 116**
**D-6800 Mannheim 31 (DE)**

(72) Erfinder: **Frey, Günther**
**Pfalzgrafenstrasse 7**
**D-6701 Ellerstadt (DE)**
Erfinder: **Zimmermann, Gerd, Dr. rer. nat.**
**Dornheimer Ring 4**
**D-6800 Mannheim 41 (DE)**

(56) Entgegenhaltungen:
**CHEMICAL ABSTRACTS, Band 77, Nr. 11, 11. September 1972, Seite 483, Zusammenfassung Nr. 75270m, Columbus, Ohio, US; B.E. IVANOV et al.: "Interaction of Mannich bases with trialkyl phosphites", & KHIM. PRIMEN. FOSFORORG. SOEDIN., TR. VSES. KONF., 3rd 1965 (Pub. 1972), 114-22**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 175 250 B1

(56) Entgegenhaltungen:

CHEMICAL ABSTRACTS, Band 73, Nr. 5, 3. August 1970, Seite 382, Zusammenfassung Nr. 25586r, Columbus, Ohio, US; N.S. KOZLOV et al.: "Properties of diphenyl esters of N-aryl-substituted aminophosphonic acids", & VESTSI AKAD. NAVUK BELARUS. SSR, SER. KHIM. NAVUK 1970, (2), 102-4

CHEMICAL ABSTRACTS, Band 85, Nr. 5, 2. August 1976, Seite 411, Zusammenfassung Nr. 33126d, Columbus, Ohio, US; H. GROSS et al.: "Alpha-substituted phosphonates. XIX. Derivatives of aminomethanebisphosphonic acid", & J. PRAKT. CHEM. 1976, 318 (1), 116-26

CHEMICAL ABSTRACTS, Band 95, Nr. 5, 3. August 1981, Seite 213, Zusammenfassung Nr. 36957t, Columbus, Ohio, US; P.M. FREDERICKS et al.: "Synthesis and biological activity of aminomethylphosphonic acids related to the herbicide glyphosate", & Z. NATURFORSCH. C: BIOSCI. 1981, 36(3-4), 242-5

CHEMICAL ABSTRACTS, Band 95, Nr. 3, 20. Juli 1981, Seite 591, Zusammenfassung Nr. 23877s, Columbus, Ohio, US; G. BIDAN et al.: "Electrochemical study of N,N-dimethylmesidine in organic medium. Electrochemical oxidation in the presence of diethyl phosphonates", & ELECTROCHIM. ACTA 1981, 26(2), 275-82

CHEMICAL ABSTRACTS, Band 92, Nr. 17, 28. April 1980, Seite 605, Zusammenfassung Nr. 146905v, Columbus, Ohio, US; & JP-A-79 135 724 (NISSAN CHEMICAL INDUSTRIES LTD.) 22-10-1979

CHEMICAL ABSTRACTS, Band 101, Nr. 7, 13. August 1984, Seite 632, Zusammenfassung Nr. 55192y, Columbus, Ohio, US; K. ISSLEIB et al.: "Synthesis of 1-aminoalkanephosphonic acids via mono- and bis(trimethylsilyl) phosphite", & Z. CHEM. 1983, 23(12), 434-6

CHEMICAL ABSTRACTS, Band 94, Nr. 26, 29. Juni 1981, Seite 609, Zusammenfassung Nr. 217487q, Columbus, Ohio, US; J.M. MANGUS et al.: "Stabilized photographic developing compositions", & RES. DISCL. 1981, 204,149

CHEMICAL ABSTRACTS, Band 94, Nr. 26, 29. Juni 1981, Seite 528, Zusammenfassung Nr. 216452n, Columbus, Ohio, US; A. MARSHALL: "An investigation into the mechanism of inhibition of a synergistic dianodic corrosion inhibitor", & CORROSION (HOUSTON) 1981, 37(4),214-22

TETRAHEDRON, Band 37, Nr. 12, 1981, Seiten 2297-2301, Pergamon Press Ltd., Oxford, GB; G. BIDAN et al.: "Utilisation en synthese du cation iminium genere in situ par oxydation electrochimique d'amines tertiaires"

**Beschreibung**

Die Erfindung betrifft Mittel und Verfahren zum Nachweis von Redox-Systemen durch oxidative Kupplung von Chromogenen.

Sowohl für die analytische Chemie, als auch für die medizinische Diagnostic, ist der Nachweis von Wasserstoffperoxid mittels geeigneten chromogenen Substanzen, unter Katalyse von Peroxidase oder peroxidatisch wirksamen Substanzen, von sehr großer Bedeutung. Dies gilt vor allem für die zahlreischen Nachweisverfahren, bei denen Wasserstoffperoxid als Zwischenprodukt der Reaktion eines Substrates mit der entsprechenden Substratoxidase und Sauerstoff gebildet und im Anschluß in Anwesenheit geeigneter chromogener Substanzen, vorwiegend in Gegenwart einer Peroxidase (POD) als Katalysator, in eine Verbindung überführt wird, die optisch erfaßbar ist und in einer quantitativen Beziehung zum gebildeten Wasserstoffperoxid steht. Weiterhin wird peroxidase häufig als Enzym-Marker in Immuntesten verwendet und durch Zusatz von Wasserstoffperoxid und obigen chromogenen Substanzen nachgewiesen.

Als Beispiele sind nachfolgend Verbindungen genannt, die mit den in Klammer stehenden entsprechenden Oxidasen wasserstoffperoxidbildende Systeme darstellen:

Glucose (Glucoseoxidase), Galactose (Galactoseoxidase), L-Aminosäure (L-Aminosäureoxidase), Cholesterin (Cholesterinoxidase), Harnsäure (Uricase), Sarcosin (Sarcosinoxidase), Glycerin (Glycerinoxidase), Glycerinphosphat (Glycerinphosphatoxidase), Pyruvat (Pyruvatoxidase).

Für den Nachweis von Wasserstoffperoxid/Peroxidase sind bereits zahlreiche Chromogene bzw. Indikatorsysteme genannt und eingesetzt worden. Eines der bekanntesten ist das von Trinder (Ann, Clin. Biochem. 6 (1969), 24—27) beschriebene Indikatorsystem, bei dem Phenol mit 4-Aminoantipyrin in Gegenwart von POD unter Einwirkung von $H_2O_2$ oxidativ zu einem Farbstoff gekuppelt wird. Dabei können anstelle des Phenols als Kupplungskomponente auch Phenolderivate, Anilinderivate, Naphthole, Naphtholderivate, Naphthylamin, Naphthylaminderivate oder ähnlich reagierende Substanzen treten. 4-Aminoantipyrin als Kupplungspartner kann z.B. durch Aminoantipyrinderivate, Vanilindiaminsulfonsäure, Methylbenzthiazolinonhydrazon (MBTH), sulfoniertes Methylbenzthiazolinonhydrazon (SMBTH), substituiert werden.

Die oxidative Kupplung wird ferner eingesetzt, um z.B bei enzymatischen Reaktionen freigesetzte aromatische Amine zu bestimmen. Für diagnostische Zwecke von besonderer Bedeutung sind der Nachweis von γ-Glutamyltranspeptidase (γGT) und Leucinaminopeptidease (LAP) sowie der Nachweis von Thrombin. In diesen Fällen wird ein "Peptidamid", dessen Aminosäuresequenz dem jeweiligen Enzym entspricht und dessen Amidteil ein aromatisches Amin, insbesondere Phenylendiamin oder Aminophenol oder ein Derivat davon ist, welches als Kupplungspartner der vorstehenden Reaktion dienen kann, zunächst durch das Enzym gespalten und dann mit einem Phenol oder Anilin als Kupplungskomponente durch ein Oxidationsmittel zum Farbstoff oxidiert (vtl. DE—A 33 31 588 und EP—A 76 042).

Diese Nachweisreaktionen können sowohl in der Küvette als auch auf Trockenreagenzträgern durchgeführt werden. Ihre Quantifizierung erfolgt dabei mittels Photometern über eine Transmissionsmessung, mit Remissionsphotometern über Remissionsmessung oder mit Hilfe von Vergleichsvarben durch visuellen Vergleich.

In der Literatur wurden mehrfach Anilinderivate beschrieben, mit denen empfindliche Nachweissysteme für Wasserstoffperoxid durch Kondensation von 4-Aminoantipyrin bzw. substituierten Benzothiazolinonhydrazonen hergestellt werden können (DE—C—2833612, EP—A—7787, DE—A—3037342). Die so gebildeten Kupplungsprodukte weisen einen hohen molaren Extinktionskoeffizienten auf, der sie zum Nachweis entsprechender wasserstoffeproxidbildender Verbindungen als gut geeignet erscheinen läßt. Nachteilig erwies sich die hohe Störanfälligkeit dieser Kupplungsprodukte gegenüber Serumbestandteilen, die Extinktionsdifferenzen gegen rein wässrige LÖsungen von über 30% ergeben sowie teilweise eine ungenügende Stabilität der Verbindungen oder der Kupplungsprodukte.

Die Aufgabenstellung der vorliegenden Erfindung war daher die Suche nach Phenol- oder Anilinderivaten als Farbbildner bei der oxidativen Kupplung insbesondere für den Nachweis von Wasserstoffperoxid bzw. peroxidatisch wirksamen Substanzen und aromatischen, kupplungsfähigen Aminen, die mit den im Serum enthaltenen Störsubstanzen nicht reagieren, im schwach sauren bis schwach alkalischen Bereich stabil sind und somit sowohl in Küvettentests als auch in allen für Trockenreagenzträger brauchbaren Matrices verwendet werden können. Überraschenderweise wurden in den Anilinderivaten der allgemeinen Formel I Verbindungen gefunden, die den erwähnten Anforderungen entsprachen.

Gegenstand der Erfindung ist die Verwendung von Anilinderivaten der allgemeinen Formel I,

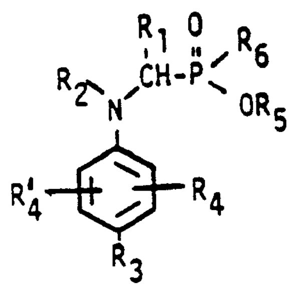

in der

$R_1$ Wasserstoff, Alkyl mit 1—6 C-Atomen, Phenyl, das zusätzlich non durch Alkyl oder Alkoxy mit 1—6 C-Atomen oder Halogen substituiert sein kann oder Naphthyl, das gegebenenfalls teilhydriert sein kann, darstellt

$R_2$ für Wasserstoff, Alkyl mit 1—6 C-Atomen, das durch Hydroxy-, Amino-, Carboxy-, $C_1$- bis $C_6$-Alkoxycarbonyl-, $C_1$- bis $C_6$-Alkanoylamidogruppen, Phenyl- und Naphthyl-($C_1$- bis $C_6$-)alkyl, wobei Phenyl noch zusätzlich durch Alkyl oder Alkoxy mit 1 bis 6 C-Atomen oder Halogen substituiert sein und Naphthyl gegebenenfalls teilhydriert sein kann und Phenyl, das zusätzlich noch durch Alkyl oder Alkoxy mit 1 bis 6 C-Atomen oder Halogen substituiert sein kann oder Naphthyl, das gegebenenfalls teilhydriert sein kann und Gruppen der Struktur

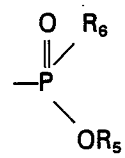

in denen $R_5$ und $R_6$ die unten angegebene Bedeutung haben, substituiert sein kann, steht,

$R_3$ Wasserstoff, Carboxy oder Halogen sein kann,

$R_4$ und $R'_4$ für Wasserstoff, Halogen, Carboxy-, $C_1$- bis $C_6$-Alkoxy- oder eine bevorzugt n m-Stellung stehende $C_1$- bis $C_6$-Alkylgruppe steht und

$R_1$ und $R_2$ bzw. $R_2$ und $R_4$ bzw. $R_4$ und $R'_4$ für den Fall, daß die beiden Substituenten orthoständig zueinander stehen, zusammen eine gesättigte oder ungesättigte Kohlenwasserstoffkette mit 2—6 C-Atomen bilden können, die durch Hydroxy- oder Oxogruppen substituiert sein kann,

$R_5$ Wasserstoff oder eine Alkylgruppe mit 1—6 C-Atomen sein kann und

$R_6$ für eine Hydroxy-, eine $C_1$- bis $C_6$-Alkoxy-, eine $C_1$- bis $C_6$-Alkylgruppe oder Phenyl, das zusätzlich noch durch Alkyl oder Alkoxy mit 1 bis 6 C-Atomen oder Halogen substituiert sein kann oder Naphthyl, das gegebenenfalls teilhydriert sein kann, steht,

sowie ihre Salze mit Säuren oder Basen als Farbbildner in Trinderreaktionen sowie Mittel, die diese enthalten.

Der in der Beschreibung verwendete Ausdruck "Niederalkyl" bedeutet geradkettige oder verzweigte Alkylreste mit 1—6, vorzugsweise 1—3 C-Atomen. Beispiele dafür sind die Methyl-, Ethyl-, Isopropyl-, Isobutyl oder die tert.Butylgruppe.

Der Ausdruck "Aryl" bezeichnet vorzugsweise die Phenylgruppe, die zusätzlich noch durch Niederalkyl oder Alkoxy oder Halogen substituiert sein kann, und die Naphthylgruppe, die ggf. teilhydriert sein kann.

Halogen soll Fluor, Chlor, Brom und Iod umfassen, wobei Fluor und Chlor bevorzugt sind.

$R_1$ und $R_2$ bzw. $R_2$ und $R_4$ bzw. $R'_4$ bzw. $R'_4$ können zusammen eine gesättigte oder ungesättigte Kohlenwasserstoffkette bilden. Eine derartige Gruppe kann 2—6, vorzugsweise 2—4 C-Atome umfassen. Beispiele dafür sind: $—(CH_2)_2—$, $—(CH_2)_3—$, $—CH{=}CH—$, $CH_2—CH{=}CH—$. Beispiele für eine durch Hydroxy- oder Oxogruppen substituierte, gesättigte oder ungesättigte Kohlenwasserstoffkette sind $—CH(OH)—(CH_2)_2—$, $CO—(CH_2)_2—CH{=}CH—C{=}O$.

Niederalkanoylreste sind Reste aliphatischer Carbonsäuren mit 1—6 C-Atomen z.B. Formyl, Acetyl, Propionyl, Pivaloyl, Isobutyryl. Niederalkoxygruppen sind Reste, die 1—6, bevorzugt 1—4 C-Atome enthalten z.B. Methoxy, Ethoxy, Propoxy, Isopropoxy, tert.-Butoxy.

Die Verbindungen der allgemeinen Formel I sind, vor allem für $R_5$ = Niederalkyl und $R_6$ = Niederalkoxy, zum Teil beschrieben (z.B. Zh. Obshch. Khim. 47, 2741 (1977), Izv. Akad. Nauk SSSR, Ser. Khim. 424 (1967), ebenda 178 (1982), US—A—3816428, Z. Naturforsch. 36c, 242 (1981), C.A. 99: 22914k, C.A. 91: 157812t, Bull. Soc. Chim. Fr. Pt. 2,343 (1979), Tetrahedron 37, 2297 (1981), J. Amer. Chem. Soc. 74, 1528 (1952), CS—A—190240). Die dort beschriebenen Verbindungen fanden z.B. Verwendung als Wachstumsregulatoren für Pflanzen, Flotationshilfsmittel, Chelatbildner und Korrosionsinhibitoren. Die Verwendung als Komponente in oxidativen Kupplungsreaktionen zum kolorimetrischen Nachweis von $H_2O_2$ bzw. Peroxidasen oder peroxidatisch wirkenden Verbindungen wurde bischer nicht beschrieben.

Weiterhin sind Gegenstand der Erfindung die neuen Verbindungen der Formel I'

I'

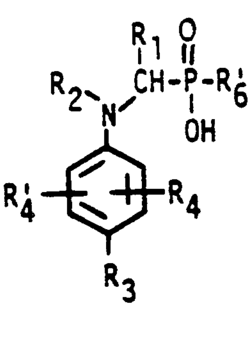

in der $R_1$, $R_3$, $R_4$ und $R'_4$ die gleiche Bedeutung haben wie in Formel I, $R_2$ für Wasserstoff, Alkyl mit 1—6 C-Atomen, das durch Hydroxy-, Amino-, Carboxy-, $C_1$- bis $C_6$-Alkoxycarbonyl-, $C_1$- bis $C_6$-Alkanoylamidogruppen, Phenyl- und Naphthyl-($C_1$- bis $C_6$-)alkyl, wobei Phenyl noch zusätzlich durch Alkyl oder Alkoxy mit 1 bis 6 C-Atomen oder Halogen substituiert sein und Naphthyl gegebenenfalls teilhydriert sein kann und Phenyl, das zusätzlich noch durch Alkyl oder Alkoxy mit 1 bis 6 C-Atomen oder Halogen substituiert sein kann oder Naphthyl, das gegebenenfalls teilhydriert sein kann und Gruppen der Struktur

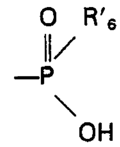

in denen $R'_6$ die nachstehende Bedeutung hat, substituiert sein kann, und $R'_6$ eine Hydroxy-, $C_1$- bis $C_6$-Alkylgruppe oder Phenyl, das zusätzlich noch durch Alkyl oder Alkoxy mit 1 bis 6 C-Atomen oder Halogen substituiert sein kann oder Naphthyl, das gegebenenfalls teilhydriert sein kann, bedeutet, sowie ihre Salze mit Säuren oder Basen, und ihre Herstellung nach an sich bekannten Methoden.

Die vorstehenden Verbindungen der Formel I und I' sind stabile Substanzen, die entweder selbst gut wasserlöslich sind oder sich durch Umsetzen mit üblichen Säuren (Mineralsäuren wie Salzsäure, Schwefelsäure, Phosphorsäure etc. oder organischen Säuren wie Essigsäure, Citronensäure, Oxalsäure etc.) oder Basen (Natronlauge, Kalilauge, Ammoniak, Aminen, Alkalicarbonaten etc.) in gut lösliche Salze überführen lassen.

Die Synthese der Verbindungen der allgemeinen Formel I kann nach an sich bekannten Methoden erfolgen. Eine allgemeine Zusammenfassung der möglichen Reaktionen zur Herstellung von > N—C—P-Systemen findet sich in Topics in Phosphorus Chemistry Vol. 8, S. 515f. (1976). Im folgenden werden einezelne Beispiele aufgehührt, die für die Synthese der Verbindungen der Formel I besonders brauchbar sind.

1. Wie z.B. in J. Amer. Chem. Soc. 74, 1528 (1952) beschrieben, kann man ein primäres oder sekundäres Amin mit einem Aldehyd und einem Dialkylphosphit oder einem Phosphonigsäuredialkylester zu einer Verbindung der Formel I umsetzen.

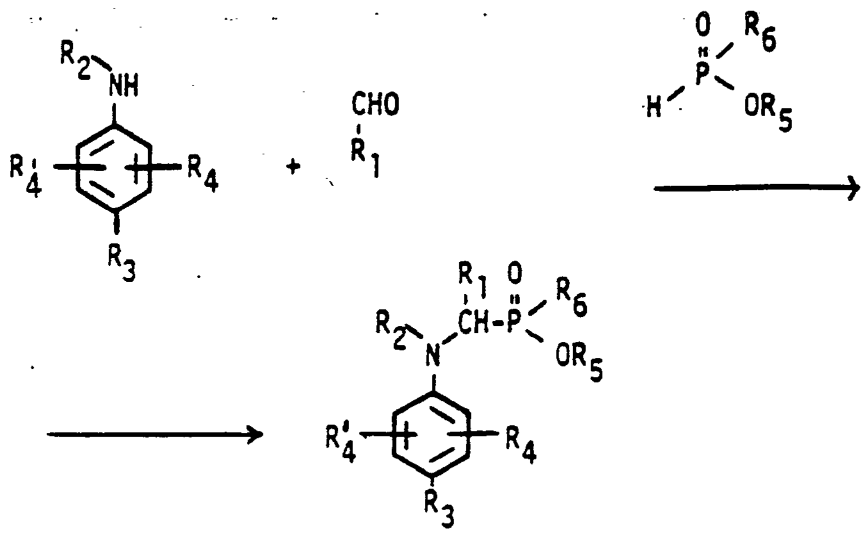

Statt der Ausgangsprodukte primäres Amin und Aldehyd lassen sich auch die daraus herstellbaren Schiffschen Basen einsetzen.

2. N,O-Acetale der Struktur II mit $R_7$ = Niederalkyl setzen sich unter $H^+$-Katalyse mit Trialkylphosphiten (III, $R_6$ = Niederalkoxy) unter Bildung von Phosphonsäureestern der Struktur I mit $R_8$ = H um (Izv. Akad. Nauk SSSR, Ser. Khim. 424 (1967).

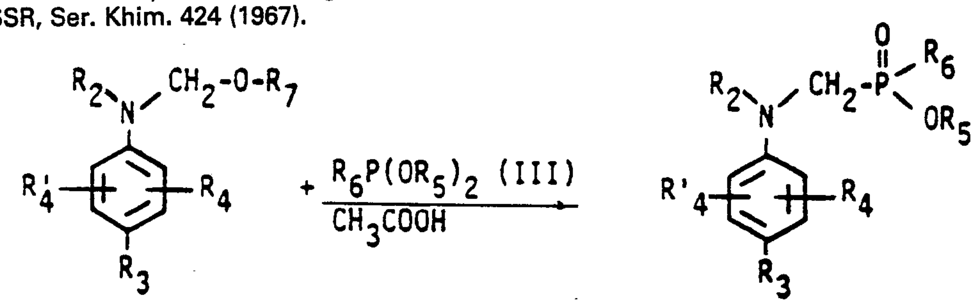


II

Diese Reaktion läßt sich auch mit Phosphonigsäuredialkylestern (III, $R_6$ = Niederalkyl oder Aryl) durchführen.

In gleicher Weise lassen sich auch cyclische N,O-Acetale z.B. N-Aryloxazolidine (IV) mit Trialkylphosphiten bzw. Phosphonigsäuredialkylestern umsetzen. Dabei können sich, besonders ausgehend von Oxazolidinen, unter intramolekularer Umesterung cyclische Ester von Phosphon- bzw. Phosphinsäuren bilden.

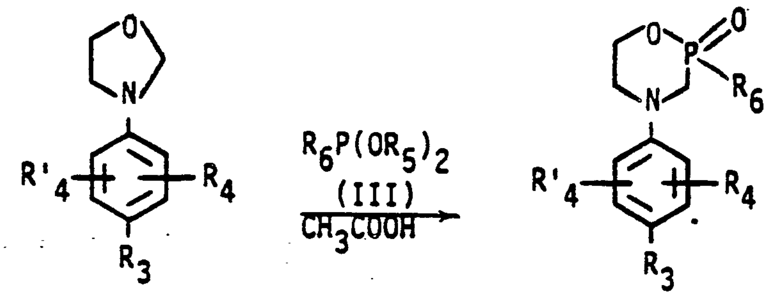


IV

Die als Ausgangsprodukt benötigten N,O-Acetale lassen sich in Analogie zu beschriebenen Reaktionen (J. Amer. Chem. Soc. 54, 4176 (1932)) durch Umsetzung von sekundären Anilinen mit Formaldehyd und alkoholen erhalten. Kann man als Ausgangsmaterial N,N-Dimethylaniline verwenden, so lassen sich die entsprechenden N,O-Acetale des Formaldehyds, wie in J. Org. Chem. 31, 4058k (1966) beschrieben, durch anodische Oxidation in methanolischer KOH herstellen. Bei entsprechend langer Elektrolysedauer sind auch Bis-N,O-Acetale der folgenden allgemeinen Struktur zugänglich, die sich mit

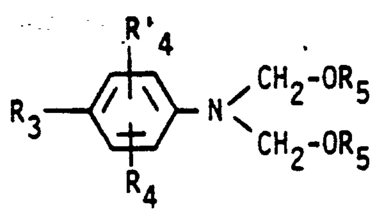

2 Mol Trialkylphosphit bzw. Phosphonigsäuredialkylester zu den entsprechenden Iminodimethanphosphonsäuren umsetzen lassen.

Cyclische N,O-Acetale lassen sich analog den offenkettigen Verbindungen herstellen. Beispielsweise erhält man N-Aryloxazolidine durch Umsetzung von N-Hydroxyethylanilinen mit Aldehyden z.B. Formaldehyd.

3. Literaturbekannt ist auch die Alkylierung von Anilinen mit PHosphonsäureestern der Struktur V, wobei χ die Bedeutung Br, J, Tosyloxy, Benzolsulfonyloxy, Methansulfonyloxy haben kann. J. Recherches C.N.R.S. 119.(1956), Zh. Obschch Khim. 47, 2741 (1977).

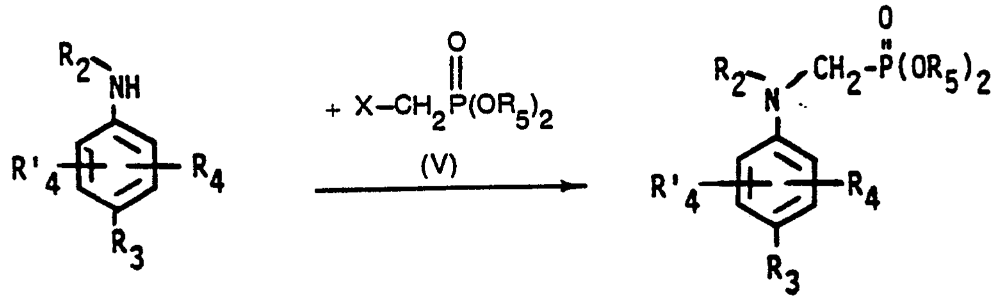


I

4. Aminoalkylphosphonsäureester der Allgemeinen Formel I, $R_5$ = Niederalkyl, $R_6$ = Niederalkoxy, bevorzugt mit $R_1$ = H, lassen sich mit starken Basen, z.B. n-Butyllithium, am Alpha-C-Atom metallieren und mit Alkylierungsmittel wie z.B. Methyljodid alkylieren (Synthesis 336, (1977))

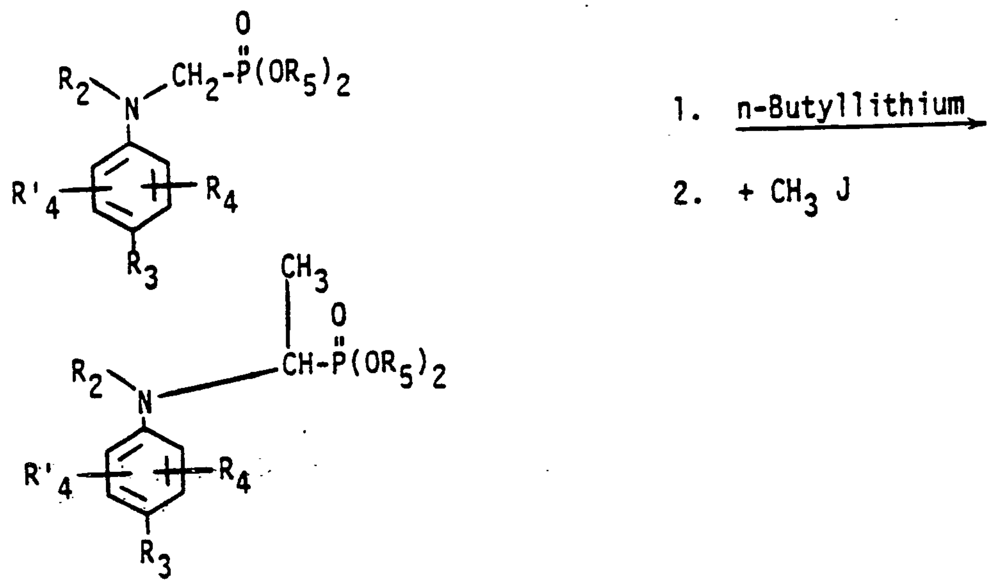


Die Verbindungen der Formel I mit $R_5$ = Niederalkyl und $R_6$ = Niederalkyl und Niederalkoxy lassen sich durch Einwirkung von konzentrierten Mineralsäuren in der Siedehitze z.B. HCl oder durch Umsetzung mit Trimethylsilylhalogeniden, besonders Trimethylsilylbromid oder -jodid, und anschließender Behandlung mit Wasser verseifen. Durch Behandeln mit Natriumalkoholat oder Alkalimetallsalzen wie Natriumjodid lassen sich aus Verbindungen der Formel I mit $R_5$ = Niederalkyl und $R_6$ = Niederalkoxy nach an sich bekannten Methoden die entsprechenden Phosphonsäurehalbester herstellen.

Die Verbindungen der Formel I können in Form der freien Basen oder als Salze starker Säuren z.B. HCl, $HBF_4$, HBr isoliert werden. Für $R_5$ = H lassen sich die Verbindungen der Formel I auch als innere Salze, Alkalimetall- oder Ammonium- bzw. Aminsalze fassen.

Die Erfindung ist durch die Ansprüche näher gekennzeichnet.

Die erfindungsgemäßen Anilinderivate liegen normalerweise als innere Salze vor, da sie sowohl saure als auch basische Gruppen enthalten.

Diejenigen Verbindungen, die in der 3-Stellung zum Aminstickstoff eine Alkylgruppe, insbesondere eine Methylgruppe enthalten, werden bevorzugt, da sie eine erhöhte Extinktion aufweisen.

Als oxidationsfähige Kupplungspartner für den Nachweis von $H_2O_2$/POD werden 4-Aminoantipyrin (4-AAP), 2-Hydrazono-2,3-dihydro-3-methylbenzthiazolon (MHTH) und insbesondere die 2-Hydrazono-2,3-dihydro-3-methylbenzthiazolon-sulfonsäure-6 (SMBTH) bevorzugt, jedoch können auch andere für ihre oxidative Kupplungsfähigkeit bekannte Substanzen in gleicher Weise eingesetzt werden.

Für den Nachweis von aromatischen Aminen, d.h. insbesondere von p-Phenylendiamin- oder p-Aminophenolderivaten, erweisen sich diese Anilinderivate der Formel I ebenfalls hervorragend geeignet, da sie störunanfällig sind und bei der oxidativen Kupplung Farbstoffe guter Stabilität und hoher Extinktion ergeben. Die für den Enzymnachweis geeigneten Substrate entsprechenden der Formel VII

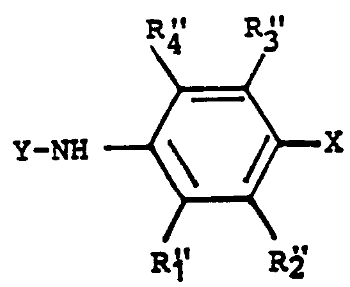

VII

in der $R''_1$—$R''_4$ Wasserstoff, Halogen, Alkyl mit 1—6 C-Atomen Alkoxy mit 1—6 C-Atomen, eine Carboxy- oder Sulfonylgruppe oder $R''_1$ und $R''_2$ oder $R''_3$ und $R''_4$ auch eine Alkyl- oder Alkylenbrücke bilden können,

X' eine Hydroxygruppe oder eine Aminogruppe darstellt, die ein- oder zweifach durch Alkyl substituiert sein kann und

Y eine gegebenenfalls geschützte Aminosäure oder Peptidgruppe darstellt.

Für den Nachweis von Leucylaminopeptidase ist Y L-Leucyl, für den Nachweis von γGT γ-L-Glutamyl und für den Nachweis von Thrombin z.B. Tosyl-Gly-Pro-Arg-.

Als Oxidationsmittel kommen außer $H_2O_2$/POD auch Peroxide wie Persulfat oder Peracetat sowie

Perjodat, Chloramin T und insbesondere Cyanoferri-Komplexe, bspw. $K_3$ $Fe(CN)_6$ in Frage. gemäß DE—A 33 31 588 lassen sich auch Oxidasen einsetzen.

Wie bereits erwähnt, wird das erfindungsgemäße System zum Nachweis von Wasserstoffperoxid bzw. Waserstoffperoxid-produzierenden Systemen sowie von Pertoxidase oder peroxidatisch wirksamen Verbindungen eingesezt. Unter Wasserstoffperoxid-produzierenden Systemen sind insbesondere die in der klinischen Diagnostik wichtigen Substrat-/Substratoxidasepaare zu verstehen, bei denen das Substrat in Gegenwart von Luftsauerstoff oxidiert und $H_2O_2$ produziert wird. Es lassen sich somit entweder Substrat oder die Substratoxidasen nachweisen, je nach dem welche Komponente dem Reagenz zugeführt wird. Ferner können die erfindungsgemäßen Substanzen zum Nachweis kupplungsfähiger, aromatischer Amine dienen, wenn sie in Gegenwart eines Oxidationsmittels mit diesen in Berührung gebracht werden. Als Beispiele wird auf die Systeme, die in der Einleitung genannt sind, verwiesen.

Es lassen sich mit den Reagenzkombinationen Tests herstellen, die in der Küvette vermessen werden. Dazu wird eine der erfindungsgemäßen Substanzen der Formel I zusammen mit den für den jeweiligen Parameternachweis notwendigen Enzymen oder anderen Reagenzien, dem Puffer, sowie gegebenenfalls Netzmitteln, Aktivatoren und anderen Hilfsstoffen als Pulver vermischt oder zu Tabletten verpreßt oder vorzugsweise in Wasser gelöst und wieder eingetrocknet oder lyophilisiert. Die so gewonnene Reagenzmischung wird vor Gebrauch in Wasser oder einem anderen geeigneten Lösungsmittel gelöst und so die Reagenzlösung bereitet. Nach Vermischen der Probe (Substratlösung, Enzymlösung, Serum oder Plasma) mit einem aliquoten Teil der Reagenzmischung wird die entstehende Farbe am Photometer vermessen und über den molaren Extinktionskoeffizienten und die zugesetzten Reagenz- bzw. Probevolumina die jeweilige Konzentration bzw. Subtratkonzentration berechnet. Es sind sowohl kinetische als auch Endpunktsmessungen möglich.

Ebenso können die Substanzen der Formel I in Verbindung mit dem oxidationsfähigen Kupplungspartner zusammen mit Peroxidase, dem/den für den jeweiligen Parameternachweis notwendigen Reagenzien bzw. anderen Enzymen, dem Puffersystem, gegebenenfalls Netzmitteln und Aktivatoren sowie anderen Hilfsstoffen auf saugfähigen Reagenzträgern wie Papieren, Vliesen etc. imprägniert werden. Dazu kann man eine oder mehrere Imprägnierlösungen in Form von wässrigen oder organischen bzw. gemischten Lösungen herstellen, je nach dem wie sich die Reagenzien oder Hilfsstoffe lösen. Mit diesen Lösungen werden saugfähige oder quellfähige Träger, vorzugsweise Filterpapier oder saugfähige Glas- oder Kunststoffvliese, imprägniert oder besprüht. Anschließend wird getrocknet. Die so hergestellten Reagenzträger können entweder als Schnelldiagnostica zur direkten Bestimmung von Inhaltsstoffen von Flüssigkeiten (z.B. in Körperflüssigkeiten wie Blut, Urin oder Speichel, oder in Lebensmitteln z.B. Fruchtsäfnet, Milch o.a.) eingesetzt werden. Die Flüssigkeit wird dabei direkt auf den Reagenzträger gebracht oder dieser kurz in die Flüssigkeit eingetaucht. Eine semiquantitative Bestimmung ist möglich, indem man die so entstandene Farbe einer Vergleichsfarbe zuordnet. Eine quantitive Auswertung läßt sich remissionsphotometrisch durchführen. Durch Eluieren der vorweggenannten Reagenzien mit Wasser bzw. Puffer oder Serum aus dem saugfähigen Träger laßt sich eine Reagenzlösung herstellen, mit der man wie oben beschrieben Substrate oder Enzyme in der Küvette am Photometer bestimmen kann. Die quantitative Bestimmung durch remissionsphotometrische Auswertung läßt sich besonders gut durchführen, wenn der Indikator gemeinsam mit den restlichen notwendigen Reagenzien und Hilfsstoffen und einem filmbildenden Kunststoff z.B. gemäß DE—C—1598153 zu einem Reagenzfilm verarbeitet wird. Die glatte Oberfläche eines solchen Films ergibt dabei weniger Störungen der Remission und eine homogenere Färbung als die üblicherweise verwendeten saugfähigen Papiere.

Puffer im Sinne dieser Erfindung haben einen pH-Wert von 6—10, insbesondere 6,5—7,5. Phosphat-, Citrat-, Borat-, GOOD-Puffer mit Alkali- oder Ammoniumgegenionen werden am häufigsten verwendet, andere Systeme sind jedoch ebenfalls brauchbar.

Netzmittel sind insbesondere anionische und kationische Netzmittel, die mit den Zwitterionischen erfindungsgemäßen Verbindungen ionische Wechselwirkungen eingehen. Nichtionogene Netzmittel, die die Enzyme aktivieren, sind jedoch ebenfalls brauchbar. Natriumlaurylsulfat, Dioctylnatriumsulfosuccinat und Alkylarylpolyetheralkohole werden bevorzugt.

Als Aktivatoren sind die für die jeweilige Substratreaktion bekannten einzusetzen. Die Trinderreaktion selbst ist so schnell, daß deine zusätzliche Aktvierung nicht notwendig erscheint.

Als sonstige Hilfsstoffe können übliche Verdicker, Emulgatoren, optische Aufheller, Kontrastmittel etc. sinnvoll sein, wie sie in entsprechenden Testen mit anderen Chromogenen bekannt sind.

Die Kupplungsreaktion erfolgt üblicherweise bei Raumtemperatur, kann jedoch auch ohne weiteres bei höherer Temperatur, beispielsweise bei 37°C, durchgeführt werden, wenn dies für die Reaktionsgeschwindigkeit beispielsweise einer vorgeschalteten enzymatischen Reaktion sinnvoll ist.

Für die üblicherweise vorkommenden Reaktionen mit Substraten bzw. Enzymen haben sich folgende Konzentrationen der Testlösung bewährt:

| | |
|---|---|
| Anilinderivat | 0,05—100 mmol/l<br>vorzugsweise 0,1—1 mmol/l |
| Kupplungspartner | 0,05—50 mmol/l<br>vorzugsweise 0,1—1 mmol/l |

| | |
|---|---|
| Puffer pH 6—10 | 0,05—1 Mol/l |
| vorzugsweise pH 6,5—8 | vorzugsweise 0,1—0,5—0,5 Mol/l |
| Netzmittel | 0—1,0 Mol/l |
| | vorzugsweise 0,05—0,1 Mol/l |
| a, Peroxidase | 1,0—5000 KU/l |
| b, $H_2O_2$ bzw. $H_2O_2$-produzierendes Substrat/Enzymgemisch | 0,1—10 mMol/l |
| sonstige Hilfsstoffe | 0—5 Mol/l |

Die vorstehenden Konzentrationsbereiche sind so zu verstehen, daß die unteren Bereiche jeweils für die photometrischen Teste in der Küvette und die oberen Bereiche für Schnellteste bzw. Teste auf festen Trägern bevorzugt sind.

Für den Nachweis von als Amidasen wirkenden Enzymen wird in der vorstehenden Aufstellung der Kupplungspartner durch eine entsprechende Menge des Peptidamidsubstrats ersetzt und ggf. statt $H_2O_2$ eine entsprechende Menge eines anderen Oxidationsmittels verwendet.

Die nachfolgenden Beispiele dienen als Anhalt, ohne daß die Erfindung darauf beschränkt wird.

## Beispiel 1

1.1

Eine Mischung aus 21,4 g N-Methylanilin und 27,6 g Diethylphosphit wird unter Rühren bei 90°C mit 21,6 g Benzaldehyd versetzt. Die Reaktionsmischung wird 1 Stunde auf 90°C gehalten und schließlich im Vakuum zur Trockene eingeengt. Das Rohprodukt wird über 1,5 Liter Kieselgel mit Ligroin/Aceton (3:1) chromatographiert. Die produkthaltigen Fraktionen werden eingeengt und eingedampft. Man erhält 25 g (38%) öligen Alpha-(N-methylanilino)-benzyl-phosphonsäurediethylester.

1.2

3,3 g Alpha-(N-methylanilino)-benzylphosphonsäurediethylester werden in 60 ml trockenem Methylenchlorid gelöst und tropfenweise mit 7,92 ml Trimethylsilylbromid versetzt. Man läßt das Gemisch über Nacht stehen, engt dann zur Trockene ein, verrünrt den Rückstand mit Ether und saugt dann 1,85 g Rohprodukt ab. Dieses wird in Wasser und wenig HCl gelöst. Die wässrige Lösung wird mit Ether extrahiert, gekohlt und zur Trockene eingeengt. Der Rückstand wird mit Ether verrieben. Der Niederschlag wird abgesaugt und getrocknet. Man erhält 1,1 g (31%) Alpha-(N-methylanilino)benzylphosphonsäure hydrobromid vom Fp. 142 bis 146°C.

## Beispiel 2

2.1

71,6 g N,N-Dimethylanilin werden in 800 ml 2 proz. methanolischem Kaliumhydroxid gelöst und in einem Becherglas 25 Stunden bei 1,5 A elektrolysiert. Die Anode besteht aus einem Platinnetz (ca. 50 $cm^2$) und die Kathode aus einem Edelstahlnetz. Die Reaktionslösung wird zur Trockene eingedampft und der Rückstand in Ether aufgenommen. Der unlösliche Rückstand wird abfiltriert und die Etherphase eingeengt. Das verbleibende Öl wird im Vakuum destilliert. Ma erhält nach zweimaliger Destillatin über eine Vigreux-Kolonne 24,3 g (27%) N-Methoxymethyl-N-methylanilin vom Siedepunkt 88,5 bis 89°C bei 7 mm Hg.

2.2

In einem Dreihalskolben mit Rührer, Tropftrichter mit Destillationsbrücke werden 5 ml N-Methoxymethyl-N-methylanilin und 4,6 ml Trimethylphosphit unter Rühren auf 135°C (Badtemperatur) erhitzt. Man tropft innerhalb einer Stunde portionsweise 1,4 ml Essigsäure zu, rührt das Gemisch noch eine weitere Stunde und destilliert das Reaktionsgemisch im Hochvakuum. Man erhält 4,4 g (58%) öligen (N-Methylanilino)-methanphosphonsäuredimethylester vom Siedepunkt 126 bis 128°C (0,1 mm Hg).

2.3

4,2 g (N-Methylanilino)-methanphosphonsäuredimethylester werden mit 15 ml konz. HCl 4 Stunden unter Rückfluß gekocht. Man engt zur Trockene ein, löst den Rückstand in 10 ml heißem Ethanol und versetzt das etwas abgekühlte Gemisch mit Ether. Die ausgefallenen Kristalle werden abgesaugt und getrocknet. Man erhält 3,03 g (78%) (N-Methylanilino)-methanphosphonsäure-Hydrochlorid vom Fp. 166—168°C (Zersetzung).

## Beispiel 3

Analog Beispiel 2 werden die in der Tabelle 1 aufgeführten Anilinophosphonsäuren hergestellt. Tabelle 2 enthält die als Ausgangsprodukte synthetisierten N-Methoxymethyl-N-methylaniline und Tabelle 3 die daraus hergestellten Anilinophosphonsäureester.

Tabelle 1

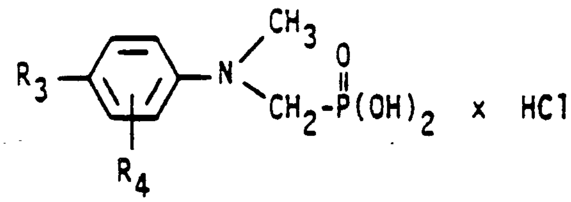

| Nr. | $R_3$ | $R_4$ | Fp. |
|---|---|---|---|
| 1 | H- | 3-Cl | 158 - 160° (Z) |
| 2 | H- | $3-CH_3$ | 186 - 188° (Z) |
| 3* | H- | $3-OCH_3$ | 128 - 129° HCl-freie Verb. |
| 4 | F- | H- | 160 - 162° (Z) |
| 5 | F- | $3-CH_3$ | 169 - 173° (Z) |
| 6 | Cl- | H- | 140 - 143° (Z) |

* Die Verbindung wird durch Chromatographie über DOWEX®50 $H^+$-Form mit Wasser gereinigt und aus Isopropanol/Wasser umkristalliert.

Tabelle 2

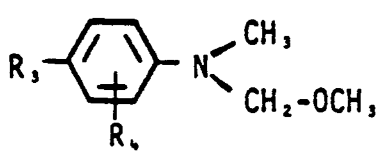

| Nr. | $R_3$ | $R_4$ | Kp |
|---|---|---|---|
| 1 | H- | 3-Cl | 98 - 103° (0,2 mm Hg) |
| 2 | H- | $3-CH_3$ | 80 - 81,5° (0,2 mm Hg) |
| 3 | H- | $3-OCH_3$ | 108 - 110° (0,2 mm Hg) |
| 4 | F- | H- | 69 - 70° (0,15 mm Hg) |
| 5 | F- | $3-CH_3$ | 108 - 115° (0,15 mm Hg) |
| 6 | Cl- | H- | 102 - 109° (1,3 mm Hg) |

Tabelle 3

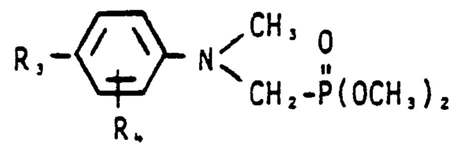

| Nr. | $R_3$ | $R_4$ | KP |
|---|---|---|---|
| 1 | H- | 3-Cl | 174 - 176° (0,3 mm Hg) |
| 2 | H- | $3-CH_3$ | 148 - 150° (0,2 mm Hg) |
| 3 | H- | $3-OCH_3$ | 176 - 177,5° (0,2 mm Hg) |
| 4 | F- | H- | 145 - 147° (0,2 mm Hg) |
| 5 | F- | $3-CH_3$ | öliges Produkt* Rf-Wert 0,55 |
| 6 | Cl- | H- | 147 - 157° (0,2 mm Hg) |

* gereinigt durch Chromatographie an Kieselgel, Laufmittel: Essigsäureethylester + 10% Ethanol

Beispiel 4

4.1

60,5 g N-Ethylanilin und 15 g Paraformaldehyd werden in 100 ml Benzol und 50 ml Ethanol gelöst und am Wasserabscheider 4 Stunden gekocht. Danach wird die Lösung zur Trockene eingedampft und der Rückstand fraktioniert destilliert. Man erhält als Hauptfraktion 49,8 g (56%) N-Ethyl-N-ethoxymethylanilin. Kp. 77—80° (0,1 mm Hg).

4.2

Analog Beispiel 2.2 setzt man N-Ethyl-N-ethoxymethylanilin mit Trimethylphosphit um. Man erhält ein Estergemisch, das neben dem überwiegend vorhandenen Dimethylphosphonsäureester durch Umesterung noch kleine Mengen Diethyl- bzw. Ethylmethylester enthält. Kp. 139,5—141,5° (0,2 mm Hg). Durch Verseifung mit konz. HCl analog Beispiel 2.3 erhält man (N-Ethylanilino)-methanphosphonsäure-Hydrochlorid vom Fp. 105—110° (Z).

Beispiel 5

Analog Beispiel 4 erhält man die in der Tabelle 4 aufgelisteten Phosphonsäuren (R = $PO(OH)_2$). Außerdem enthält die Tabelle 4 die als Zwischenprodukte hergestellten Alkoxymethylamine (R = $—CH_2—O$-Alkyl) und die daraus hergestellten Phosphonsäuredialkylester (R = $PO(O\text{-Alkyl})_2$). Einzelne Phosphonsäureester (vgl. Anmerkungen in Tab. 4) wurden analog Beispiel 1.2 mit Trimethylsilylbromid gespalten. Bei der Verbindungen 3—8 wurde Triethylphosphit verwendet.

Tabelle Beispiel 4

| Nr. | Struktur | phys. Daten |
|---|---|---|
| 1 | 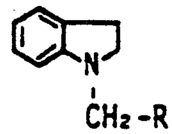<br>a) R = $-PO(OH)_2$<br>Hydrochlorid | Fp.: 156° (Z) |
| | b) R = $-PO(OCH_3)_2$ [a] | Zersetzung bei T > 110° |
| | c) R = $-OC_2H_5$ | Kp: 110-112° (0,1 mm Hg) |
| 2 | 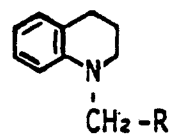<br>a) R = $-PO(OH)_2$<br>Hydrochlorid | Fp.: 170 - 174° (Z) |
| | b) R = $-PO(OCH_3)_2$ | Kp: 167-168° (0,2 mm Hg) |
| | c) R = $-OC_2H_5$ | Kp: 142-145° (4 mm Hg) |
| 3 | 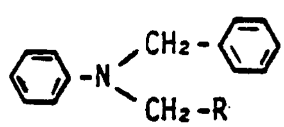<br>a) R = $-PO(OH)_2$ [b] | Fp.: 131 - 134° (Z) |
| | b) R = $-PO(OC_2H_5)_2$ | Kp: 203-205° (0,3 mm Hg) |
| | c) R = $-OC_2H_5$ | Kp: 157-161° (0,4 mm Hg) |
| 4 | 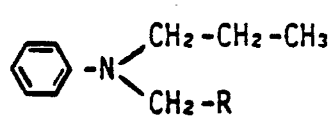<br>a) R = $-PO(OH)_2$<br>Hydrochlorid | Fp.: 72 - 75° (Z) |
| | b) R = $-PO(OC_2H_5)_2$ | Kp: 142-146° (0,1 mm Hg) |
| | c) R = $-OC_2H_5$ | Kp: 94-100° (0,15 mm Hg) |

| Nr. | Struktur | phys. Daten |
|---|---|---|
| 5 | 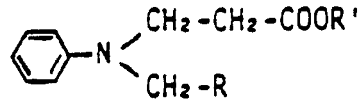 | |
| | a) R = $-PO(OH)_2$, R' = H c) | Fp.: 82 - 104° (Z) |
| | b) R = $-PO(OH)_2$, R' = $-C_2H_5$ c) | Fp.: ab 172° (Z) |
| | c) R = $-PO(OC_2H_5)_2$, R' = $-C_2H_5$ | Kp: 176-178° (0,2 mm Hg) |
| | d) R = $-OC_2H_5$, R' = $-C_2H_5$ | Kp: 128-130° (0,2 mm Hg) |
| 6 | 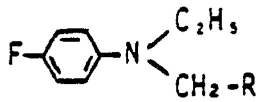 | |
| | a) R = $-PO(OH)_2$ | Fp.: 135 - 138° (Z) |
| | b) R = $-PO(OC_2H_5)_2$ | Kp: 133-137° (0,15mm Hg) |
| | c) R = $-OC_2H_5$ | Kp: 64-70° (0,15 mm Hg) |
| 7 | 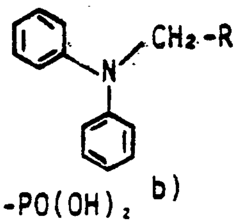 | |
| | a) R = $-PO(OH)_2$ b) | Fp.: 117 - 122° |
| | b) R = $-PO(OC_2H_5)_2$ | Kp: 183-187° (0,2 mm Hg) |
| | c) R = $-OC_2H_5$ | Kp: 133-135° (0,15mm Hg) |
| 8 | 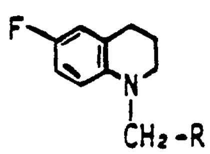 | |
| | a) R = $-PO(OH)_2$ | Fp.: 115 - 117° (Z) |
| | b) R = $-PO(OC_2H_5)_2$ | Kp: 165° (0,3 mm Hg) |
| | c) R = $-OC_2H_5$ | Kp: 103° (0,2 mm Hg) |

a) Reinigung durch Chromatographie an Kieselgel Laufmittel: Essigsäureethylester, 2—10% Ethanol

b) Diese Phosphorsäure wird aus dem entsprechenden Dialkylester durch Spaltung des Esters mit Trimethylbromsilan erhalten (analog Bsp. 1.2)

c) Diese Phosphorsäure wird durch Chromatograpie an dem stark sauren Ionenaustauscher DOWEX 50®H⁺-Form sit Wasser als Elutionsmittel gereinigt

Beispiel 6

Analog Beispiel 2 erhält man aus N,N-Dimethyl-1-naphthylamin die N-Methoxymethylverbindung, die durch Umsetzung mit Trimethylphosphit öligen N-Methyl-1-naphthylamino-methanphosphonsäure-dimethylester liefert, der durch Chromatographie an Kieselgel mit Ligroin/Aceton (3:1—3:2) gereinigt wird. Verseifung dieses Esters durch Kochen mit konz. HCl ergibt N-Methyl-1-naphthylaminomethanphosphonsäure-Hydrochlorid vom Fp. 78—80° (Z).

Beispiel 7

7.1

18 g 3-Phenyloxazolidin werden analog 2.2 mit Trimethylphosphit in Gegenwart von Eisessig umgesetzt. Das Rohprodukt wird durch Chromatographie an Kieselgel mit Ligroin/Ethanol (80:20—70:30) gereinigt. Man erhält 15 g öliges Produkt, das folgende Struktur aufweist.

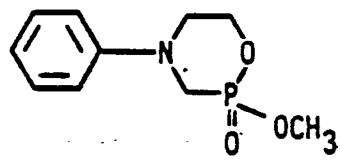

7.2

2,3 g der oben erhaltenen Verbindung werden in 60 ml trockenem Methylenchlorid gelöst und unter Rühren mit 7,9 ml Trimethylsilylbromid versetzt. Nach dem Stehen über Nacht wird die Reaktionsmischung mit 60 ml konz. $NH_3$ versetzt. Man läßt 1 Stunde nachrühren und dampft zur Trockene ein. Der Rückstand wird in Ethanol unter Zusatz von konz. HCl gelöst. Die Lösung wird mit Propylenoxid versetzt und der ausgefallene Niederschlag abgesaugt. Man erhält 0,85 g (40%) (N-(2-Aminoethyl)-anilino)-methanphosphonsäure vom Fp. 280—285° (Z).

Beispiel 8

18,3 g N-Methyl-anilino-methan-phosphonsäuredimethylester (aus Beispiel 2.2) werden in 100 ml trockenem Ether gelöst und auf −70° gekühlt. Unter Stickstoff werden 54 ml n-Butyllithiumlösung (15%) in Hexan) zugetropft. Die Reaktionsmischung wird 2 Stunden bei −70°C gerührt, mit einer Lösung von 4,9 ml Methyljodid in 20 ml Ether versetzt, weitere 2 Stunden bei −70°C gerührt, über Nacht bei Raumtemperatur stehen gelassen und mit 20 ml Wasser durchgeschüttelt. Die organische Phase wird abgetrennt, ge trocknet und eingeengt. Das verbleibende Öl wird an Kieselgel mit Essigsäureethylester/Ethanol (98:2) chromatographiert. Man erhält 2,15 g öligen 1-(N-Methylanilino)-ethan-phosphonsäuredimethylester. Dieser Ester wird analog Beispiel 1.2 mit Trimethylbromsilan gespalten. Das Rohprodukt wird über Kieselgel mit n-Propanol/Wasser/$NH_3$ (6:3:1) als Elutionsmittel chromatographiert. Die produkthaltigen Fraktionen werden vereinigt und zur Entfernung der $NH_4^+$-Ionen über den sauren Ionenaustauscher DOWEX 50 $H^+$-Form gegeben. Elution mit Wasser liefert nach dem Eindampfen 0,5 g eines glasigen Produktes, das zur Reinigung aus Aceton/Ether umkristallisiert wird. Man erhält schließlich 0,27 g 1-(N-Methylanilino)-ethanphosphonsäure vom Fp. 103—106° (Z).

Beispiel 9

3,5 g der in Beispiel 7.1 erhaltenen Verbindung werden mit 25 ml konz. HCl 5 Stunden unter Rückfluß gekocht. Das Reaktionsgemisch wird eingedampft und über Kieselgel mit n-Propanol/$H_2O$/konz. NH, (6:3:1) als Laufmittel chromatographiert. Die produkthaltigen Fraktionen werden vereinigt, eingedampft und der Rückstand zur Entfernung der $NH_4^+$-Ionen über DOWEX® 50 $H^+$-Form mit Wasser chromatographiert. Nach dem Eindampfen der entsprechenden Fraktionen verbleibt ein Öl, das nach einigen Tagen durchkristallisiert.

Man erhält 1,78 g (50%) N-Hydroxyethylanilinomethanphosphonsäure von Fp 120—121°.

Beispiel 10

N,N-(Bismethoxymethyl)-anilin werden analog Beispiel 2.2 mit 2 Mol Trimethylphosphit umgesetzt und der erhaltene Bisphosphonsäuredimethylester analog Beispiel 1.2 mit Trimethylsilylbromid verseift. Das Rohprodukt wird zur Reinigung über einen stark sauren Ionenaustauscher (DOWEX® 50 $H^+$-Form) mit Wasser chromatographiert. Durch Eindampfen der produkthaltigen Fraktionen erhält man ein Glas, das nach dem Anreiben mit einem Gemisch aus Aceton, Ether und Ligroin kristallin wird. Man erhält Phenylimino-dimethanphosphonsäure von Fp. 130—131°.

Beispiel 11

11.1

Analog Beispiel 2.2 setzt man 2,2 ml Methanphosphonigsäurediethylester mit 2 g N-Methoxymethyl-N-methylanilin bei 100° Badtemperatur um. Man erhält 1,65 g (55%) (N-Methylanilino)-methyl)-methylphosphinsäureethylester (Kp. 123° (0,1 mm Hg), der noch eine kleine Menge des entsprechenden Methylesters enthält.

11.2

2 g des oben erhaltenen Produktes erden mit 8 ml konz. Salzsäure 4 Stunden unter Rückfluß erhitzt. Die

Mischung wird zur Trockene eingedampft und der Rückstand aus Ethanol/Ether umkristallisiert. Man erhält 1,98 g (95%) (N-Methylanilino)-methyl)-methylphosphinsäure-Hydrochlorid vom Fp. 166—168° (Z).

Beispiel 12

4,5 g (N-Methylanilino)-methanphosphonsäuredimethylester werden in 20 ml Methylethylketon gelöst und mit 3 g Natriumjodid versetzt. Das Gemisch wird 30 Minuten unter Rückfluß gekocht, abgekühlt und mit Ether versetzt Der ausgefallene Niederschlag wird abfiltriert und aus Ethanol/Ether umkristallisiert. Zur weiteren Reinigung chromatographiert man das Produkt über Kieselgel mit Ethanol als Elutionsmittel. Die produkthaltigen Fraktionen werden eingedampft und der Rückstand aus Ethanol/Ether umkristallisiert. Man erhält 0,71 g (15%) (N-Methylanilino)-methanphosphonsäuremonomethylester, der sich beim Erhitzen ab 180° zersetzt.

Beispiel 13

1,5 g (N-(2-Aminoethyl)-anilino)-methanphosphonsäure (aus Beispiel 7 werden in 4 ml Wasser und 6 ml 2 N NaOH gelöst. Die Lösung wird mit wenig Eisessig versetzt, bis eine Trübung auftritt. Danach werden 2 ml Acetanhydrid zugegeben, die Mischung bei Raumtemperatur 2 Stunden gerührt und die Lösung eingeengt. Der Rückstand wird an 20 ml DOWEX® 50 $H^+$-Form mit Wasser chromatographiert. Die produkthaltigen Fraktionen werden vereinigt und eingedampft. Der Rückstand wird aus Ethanol/Ether umkristalliert. Man erhält 1,25 g (71%) (N-(2-Acetamidoethyl)-anilino-methanphosphonsäure vom Fp. 117—118° (Z).

Beispiel 14

14.1

19,85 g Succinimid, 16,2 ml wässrige Formaldehydlösung (37%) und 150 ml Ethanol werden 45 Minuten unter Rückfluß gekocht. Man läßt die Mischung etwas abkühlen, gibt dann eine Lösung von 30,2 g 3-Aminobenzoesäuremethylester in 50 ml Ethanol zu und kocht 6 Stunden unter Rückfluß. Die Reaktionslösung wird etwas eingeengt und im Kühlschrank aufbewahrt. Die ausgefallenen Kristalle werden abgesaugt. Man erhält 33,4 g 3-Succinimidomethylaminobenzoesäuremethylester, die in 90 ml Dimethylsulfoxid gelöst werden und mit 4,75 g Natriumborhydrid (Tabletten) versetzt werden. Nach Auflösung der Tablette wird die Mischung noch 20 Minuten auf 100° erhitzt. Man gießt die Mischung auf Eiswasser und extrahiert mit Ether. Die Etherphase wird mit einer Lösung von 9 g Zinkchlorid in 9 ml Wasser versetzt und kräftig gerührt. Die wässrige Phase wird abgetrennt und die Etherphase mit Wasser gewaschen. Man erhält 15,1 g öligen 3-N-Methylaminobenzoesäuremethylester.

14.2

Der oben erhaltene Benzoesäureester wird analog Beispiel 4.1 mit Paraformaldehyd und Methanol zu dem entsprechenden N,O-Acetal umgesetzt, das analog Beispiel 2.2 mit Trimethylphosphit öligen 3-(N-Dimethoxyphosphonomethyl-N-methyl)-benzoesäuremethylester ergibt, der durch Chromatographie an Kieselgel mit Essigsäureethylester/1% Ethanol als Laufmittel gereinigt wird. Verseifung dieses Esters analog Beispiel 2.3 ergibt 3-(N-Dihydroxyphosphonomethyl-N-methyl)-benzoesäure vom Fp. 189—191° (Z).

Beispiel 15

Bestimmung von $H_2O_2$ in wässriger Lösung

*Reagenzlösungen*

*Lösung A:* 0,05 mmol/l SMBTH[(1)]
0,5 mmol/l Anilinderivat
500 KU/l Peroxidase
0,1 mol/l Phosphatpuffer (pH = 8,0)

*Lösung B:* 0,05 mmol/l MBTH[(2)]
0,5 mmol/l Anilinderivat
500 KU/l Peroxidase
0,1 mol/l Phosphatpuffer (pH = 8,0)

*Lösung C:* 0,05 mmol/l 4-AAP[(3)]
0,5 mmol/l Anilinderivat
500 KU/l Peroxidase
0,1 mol/l Phosphatpuffer (pH = 8,0)

[(1)] = 3-Methyl-2-benzthiazolinon-hydrazon-6-sulfonsäure
[(2)] = 3-Methyl-2-benzthiazolinon-hydrazon
[(3)] = 4-Aminoantipyrin

In einer 1 cm-Küvette werden 600 µl Phosphatpuffer pH 8 vorgelegt und jeweils 100 µl der Anilinderivat, Peroxidase und Kuppler enthaltenden Lösung zugegeben. Nach dem Abgleichen des Leerwertes am Eppendorf-Photometer werden 100 µl einer $H_2O_2$-Stammlösung zugegeben ($CH_2O_2 \triangleq 5 \times 10^{-5}$ mol/l) und nach 2 bzw. 5 Minuten die Extinktion bei $\lambda_{max}$ gemessen. Nachstehende Extinktionen wurden ermittelt, wobei die Anilinderivate 8—10 als Vergleich mitgeführt wurden. Es zeigt sich, daß die Anilino-Phosphonsäurederivate eine 2—3-fach höhere Extinktion aufwiesen, wie andere saure Gruppen haltige Aniline, die bisher für den erfindungsgemäßen Zweck verwendet wurden.

Tabelle Beispiel 15

| Anilinderivate * | $\lambda_{max}$ | SMBTH 2' | SMBTH 5' | $\lambda_{max}$ | MBTH 2' | MBTH 5' | $\lambda_{max}$ | 4-AAP 2' | 4-AAP 5' |
|---|---|---|---|---|---|---|---|---|---|
| 1 | 570 | 1041 | 1042 | 590 | 922 | 932 | 560 | 474 | 471 |
| 2 | 570 | 1367 | 1372 | 590 | 958 | 962 | 550 | 841 | 838 |
| 3 | 560 | 911 | 911 | 560 | 973 | 972 | 550 | 294 | 293 |
| 4 | 560 | 1532 | 1516 | 560 | 1412 | 1435 | 550 | 544 | 546 |
| 5 | 570 | 1649 | 1656 | 580 | 1307 | 1319 | 560 | 790 | 795 |
| 6 | 560 | 918 | 921 | 570 | 912 | 936 | 550 | 175 | 174 |
| 7 | 560 | 1823 | 1822 | 570 | 1736 | 1729 | 557 | 510 | 508 |
| 8 | 580 | 459 | 467 | 590 | 280 | 291 | 550 | 535 | 525 |
| 9 | 590 | 480 | 501 | 590 | 377 | 408 | 550 | 517 | 510 |
| 10 | 580 | 623 | 636 | 590 | 483 | 526 | 550 | 500 | 487 |
| 11 | 600 | 121 | 122 | 610 | 58 | 58 | 550 | 237 | 226 |
| 12 | 570 | 475 | 486 | 590 | 445 | 444 | 550 | 443 | 441 |

* Die Anilinderivate sind auf Seite 36 namentlich aufgeführt.

Beispiel 16

Ermittlung der Serumstörung

*Reagenzlösungen*

*Lösung A:* 0,05 mmol/l SMBTH(1)
0,5 mmol/l Anilinderivat*
500 KU/l Peroxidase
0,1 mol/l Phosphatpuffer

*Lösung B:* 0,05 mmol/l MBTH(1)
0,5 mmol/l Anilinderivat*
500 KU/l Peroxidase
Humanserum

* = In Phosphatpuffer (0,1 mmol/l; pH = 8,0) gelöst.
(1) = 3-Methyl-2-benzthiazolon-hydrazon-6-sulfonsäure.

Wie im Beispiel 15 beschrieben werden die Extinktionsmessungen mit Lösung A durchgeführt.

Bei Lösung B wurden die vorgelegten 600 µl Phosphatpuffer durch Humanserum ersetzt.

Die in beiden Fällen ermittelten Extinktionswerte nach 2 bzw. 5 Minuten wurden in nachstehender Tabelle gegenübergestellt.

Tabelle Beispiel 16

| Anilinderivate* | $\lambda_{max}$ | (mE) mit SMBTH 2' PUFFER 5' | | (mE) mit SMBTH 2' SERUM 5' | | Δ(mE) 2' | Δ(mE) 5' | % |
|---|---|---|---|---|---|---|---|---|
| 1 | 570 | 1041 | 1042 | 963 | 961 | -78 | -81 | 7,5<br>7,8 |
| 2 | 570 | 1367 | 1372 | 1150 | 1146 | -217 | -226 | 15,8<br>16,5 |
| 3 | 560 | 911 | 911 | 895 | 898 | -16 | -13 | 1,8<br>1,4 |
| 4 | 560 | 1532 | 1516 | 1287 | 1281 | -245 | -235 | 16,0<br>15,5 |
| 6 | 560 | 918 | 921 | 808 | 802 | -110 | -119 | 12,0<br>12,9 |
| 7 | 560 | 1823 | 1822 | 1545 | 1558 | -278 | -264 | 15,2<br>14,5 |
| 8 | 580 | 459 | 467 | 127 | 131 | -332 | -336 | 72,2<br>71,5 |
| 9 | 590 | 480 | 501 | 168 | 190 | -312 | -311 | 65,0<br>62,2 |
| 10 | 580 | 623 | 636 | 404 | 413 | -219 | -223 | 35,3<br>34,8 |

* Die Anilinderivate sind auf Seite 36 namentlich aufgeführt.

Beispiel 17

Bestimmung von von Triglyceriden im Serum

In eine Filmbeschichtungsmasse werden die zum Nachweis erforderlichen Reagenzien eingeearbeitet:

| | |
|---|---|
| Phosphatpuffer pH = 8 | 0,2 mol/l |
| Detergenz (Fettalkoholpolyglykolether) | 0,5% |
| SMBTH | 0,2 mmol/l |
| (N-Ethyl-4-fluoranilino)-methanphoshons. | 1 mmol/l |
| Glycerinphosphatoxidase | 1 KU/l |
| Glycerinkinase | 3 KU/l |
| Cholesterinesterase | 3 KU/l |
| Peroxidase | 10 KU/l |

Die Beschichtungsmasse wird mit 200 µm Dicke auf eine Pokalonfolie aufgerakelt. Zur Filmbildung wird ca. 30 Minuten bei 45°C im Trockenschrank getrocknet.

Zur Bestimmung der Triglyceridkonzentration werden auf den Film 10 µl Serum aufgebracht. Nach 2—3 Minuten Reaktionszeit bei 37°C wird mit dem Photometer die Remission gemessen. Über eine vorher ermittelte Kalibrationskurve läßt sich die Triglyceridkonzentration präzise errechnen.

In der folgenden Tabelle werden die Werte für eine Eichkurve zur Ermittlung der Triglyceridkonzentrationen wiedergegeben:

| Konzentration Triglyceride (mg/dl) | Remission (%) |
|---|---|
| 100 | 64 |
| 150 | 52 |
| 200 | 41 |
| 250 | 33 |
| 300 | 27 |
| 400 | 22 |
| 500 | 18 |

Beispel 18

Bestimmung von Creatinin im Serum

Ein saugfähiger Träger (z.B. Schablonenpapier Fa. Schöller und Hösch; Flächengewicht 12 g/m$^2$; Sangfähigkeit 50 ml/m$^2$) wird mit einer Lösung bestehend aus 20 mmol/l 1,2,3,4-Tetrahydrochinolinylmethanphosphonsäure, 300 KU/l Peroxidase gelöst in Phosphatpuffer pH 7/0,2 molar imprägniert und getrocknet (Reagenzpapier A). Reagenzpapier B wird gefertigt, indem man den o.g. Träger mit der Imprägnierlösung 20 KU/l Sarcosinoxidase; 30 KU/l Creatinamidinohydrolase; 40 KU/l. Creatininamidohydrolase; 100 mmol/l sulfoniertes Methylbenzthiazolinonhydrazon (SMBTH) sowie 0,1% Detergenz (Fettalkoholpolyglykolether) in Phosphatpuffer pH 8/0,2 molar gelöst imprägniert und trocknet.

Zum Nachweis von Creatinin werden beide Papiere (A + B) in ein Testmodell eingearbeitet (Abb. 1).

Abb. 1

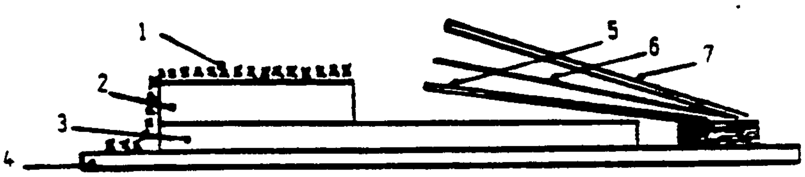


1 = Schutzgewebe
2 = Abtrennzone
3 = Transportzone
4 = Trägerfolie
5 = Reagenzträger (B)
6 = Reagenzträger (A)
7 = Abdeckfolie (transparent)

Auf die Dosierzone werden zum Nachweis von Creatinin 30 µl Serum auf die Dosierzone pipettiert. Die Reaktion wird gestartet, indem man in einem Vorgant das Indikator- und Enzympapier auf die Transportzone andrückt. Nach einer Minute Reaktionszeit (37°C) wird die gebildete Farbe remissionsphotometrisch vermessen. Über die entsprechende Eichkurve wird die zu bestimmende Creatininkonzentration ermittelt.

**Creatinin-Eichkurve**

| Serumcreatinin (mg/dl) | Remission (%) |
|---|---|
| 0,5 | 59 |
| 1,0 | 50 |
| 1,5 | 42 |
| 2,0 | 35 |
| 5,0 | 22 |
| 10,0 | 15 |

Anilinderivatbezeichnungen

1 = (N-Methylanilino)-methanphosphonsäure
2 = (4-Fluor-N-methylanilino)-methanphosphonsäure
3 = (3-Methyl-N-methylanilino)-methanphosphonsäure
4 = (4-Fluor-3-methyl-N-methylanilino)-methanphosphonsäure
5 = (N-Ethyl-4-fluoranilino)-methanphosphonsäure
6 = 1,2,3,4-Tetrahydrochinolinyl-N-methanphosphonsäure
7 = 6-Fluor-1,2,3,4-tetrahydrochinolinyl-N-methanphosphonsäure
8 = (3-Methyl-N-ethylanilino)-ethansulfonsäure
9 = 4-(N-ethyl-3-methylanilino)-methylbenzoesäure
10 = Na-bis-(3-Methylphenylimino)-propionat
11 = 4-bis-(3-Methylphenylimino)-methylbenzoesäure
12 = N-Hydroxyethyl-1,2,3,4-tetrahydrochinolin

Bestimmung der Einphasengerinnungszeit nach Quick im Photometer

In eine Küvette werden 1200/µl einer Lösung folgender Zusammensetzung pipettiert:

Tris/HCl 100 mmol/l, pH 8,1, Calciumchlorid 6 mmol/l, Tos-Gly-Pro-Arg-p-phenylendiamin, 0,1 mmol/l, N-Methyl-N-(4-methylphenyl)-methylen-phosphonsäure, 5 mmol/l, Kaninchenhirn-Thromboplastin 1,2 mg/ml.

Diese Lösung wird auf 37°C temperiert. Gleichzeitig werden je 100 µl Citratplasma und 100 µl einer wäßrigen Lösung von 10 mol/l $K_3[Fe(CN)_6]$ zupipettiert, gut gemischt und über einen gleichzeitig mit der Zugabe der Probe gestarteten Schreiber, bei einer Wellenlänge von 670 mm, die Zunahme der Extinktion nach der Zeit verfolgt.

Als Meßgröße wird die Zeit genommen, die vom Start der Reaktion bis zum Erreichen einer Extinktionsänderung von 0,1, vergeht.

Die nach dem beschriebenen Verfahren erhaltene Funktionskurve wird tabellarisch wiedergegeben.

| % Quick | Sekunden bis zum Erreichen einer Extinktionsänderung von 0,1 |
|---|---|
| 100% | 25,8 |
| 50% | 31,6 |
| 33% | 36,3 |
| 25% | 40,1 |
| 12,5% | 53,9 |
| 10% | 60,3 |

**Patentansprüche**

1. Verwendung von Anilinderivaten der Formel I

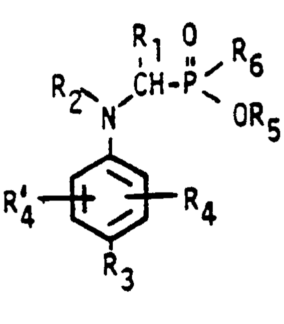

in der

$R_1$ Wasserstoff, Alkyl mit 1—6 C-Atomen, Phenyl, das zusätzlich noch durch Alkyl oder Alkoxy mit 1—6 C-Atomen oder Halogen substituiert sein kann oder Naphthyl, das gegebenenfalls teilhydriert sein kann, darstellt

$R_2$ für Wasserstoff, Alkyl mit 1—6 C-Atomen, das durch Hydroxy-, Amino-, Carboxy-, $C_1$- bis $C_6$-Alkoxycarbonyl-, $C_1$- bis $C_6$-Alkanoylamidogruppen, Phenyl- und Naphthyl-($C_1$- bis $C_6$-)alkyl, wobei Phenyl noch zusätzlich durch Alkyl oder Alkoxy mit 1 bis 6 C-Atomen oder Halogen substituiert sein und Naphthyl

gegebenenfalls teilhydriert sein kann und Phenyl, das zusätzlich noch durch Alkyl oder Alkoxy mit 1 bis 6 C-Atomen oder Halogen substituiert sein kann oder Naphthyl, das gegebenenfalls teilhydriert sein kann und Gruppen der Struktur

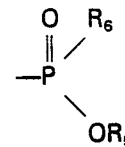

in denen $R_5$ und $R_6$ die unten angegebene Bedeutung haben, substituiert sein kann, steht,

$R_3$ Wasserstoff, Carboxy oder Halogen sein kann,

$R_4$ und $R_4'$ für Wasserstoff, Halogen, Carboxy-, $C_1$- bis $C_6$-Alkoxy-oder eine bevorzugt in m-Stellung stehende $C_1$- bis $C_6$-Alkylgruppe steht und

$R_1$ und $R_2$ bzw. $R_2$ und $R_4$ bzw. $R_4$ und $R_4'$ für den Fall, daß die beiden Substituenten orthoständig zueinander stehen, zusammen eine gesättigte oder ungesättigte Kohlenwasserstoffkette mit 2—6 C-Atomen bilden können, die durch Hydroxy- oder Oxogruppen substituiert sein kann,

$R_5$ Wasserstoff oder eine Alkylgruppe mit 1—6 C-Atomen sein kann und

$R_6$ eine Hydroxy-, eine $C_1$- bis $C_6$-Alkoxy, eine $C_1$- bis $C_6$-Alkylgruppe oder Phenyl, das zusätzlich noch durch Alkyl oder Alkoxy mit 1—6 C-Atomen oder Halogen substituiert sein kann oder Naphthyl, das gegebenenfalls teilhydriert sein kann, steht,

sowie ihre Salze mit Säuren oder Basen,

A) als Farbbildner zusammen mit einer oxidierbaren Kupplungskomponente in der Trinder-Reaktion zum Nachweis von

a) Wasserstoffperoxid oder Wasserstoffperoxid-bildenden Systemen oder

b) Peroxidase oder peroxidatisch wirkenden Substanzen oder

B) zusammen mit einem Oxidationsmittel zum Nachweis von aromatischen Aminen oder solche Amine bildenden Systemen.

2. Verwendung gemaß Anspruch 1, dadurch gekennzeichnet, daß als Kupplungskomponente 4-Aminoantipyrin oder ein Derivat desselben oder ein gegebenenfalls sulfoniertes Methylbenzthiazolinonhydrazon verwendet wird.

3. Mittel zum Nachweis von Wasserstoffperoxid oder Wasserstoffperoxid-bildenden Systemem oder Peroxidase oder peroxidatisch wirkenden Substanzen enthaltend eine Kupplungskomponente und einen Farbbildner für die Trinderreaktion und

a) eine peroxidatisch wirksame Verbindung oder

b) $H_2O_2$ oder ein $H_2O_2$ produzierendes System sowie

geeignete Puffer und ggf. Netzmittel, Aktivatoren oder andere für die Trinder-Reaktion bekannte Hilfsstoffe, dadurch gekennzeichnet, daß als Farbbildner ein Anilinderivat der Formel I enthalten ist.

4. Mittel nach Anspruch 3, dadurch gekennzeichnet, daß als Kupplungskomponente 4-Aminoantipyrin oder ein Derivat desselben oder ein gegebenenfalls sulfoniertes Methylbenzthiazolinonhydrazon verwendet wird.

5. Mittel zum Nachweis von aromatischen Aminen oder diese freisetzenden Systemen enthaltend ein Oxidationsmittel sowie geeignete Puffer und ggf. Netzmittel, Aktivatoren oder andere für die Trinder-Reaktion bekannte Hilfsstoffe, dadurch gekennzeichnet, daß als Farbbildner ein Anilinderivat der Formel I enthalten ist.

6. Mittel gemäß Anspruch 5, dadurch gekennzeichnet, daß als Oxidationsmittel $[Fe(CN)_6]^{3-}$, $H_2O_2$/POD, Perjodat oder Persulfat verwendet wird.

7. Mittel gemäß Anspruch 5 zum Nachweis einer Amidase, dadurch gekennzeichnet, daß das Mittel als Amin freisetzendes Substrat eine Verbindung der Formel VII

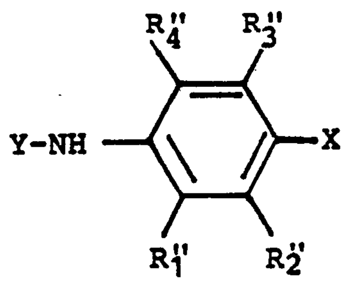

VII

in der $R''_1$—$R''_4$ Wasserstoff, Halogen, Alkyl mit 1—6 C-Atomen, Alkoxy mit 1—6 C-Atomen, eine Carboxy- oder Sulfonylgruppe oder $R''_1$ und $R''_2$ oder $R''_3$ und $R''_4$ auch eine Alkyl- oder Alkylenbrücke bilden können.

X′ eine Hydroxygruppe oder eine Aminogruppe darstellt, die ein- oder zweifach durch Alkyl substituiert sein kann und

Y eine gegebenenfalls geschützte Aminosäure oder Peptidgruppe darstellt, enthält.

8. Mittel zur Bestimmung der Einphasengerinnungszeit nach Quick, bestehend aus Tos-Gly-Pro-Arg-p-Phenylendiamin, Puffer, $K_3[Fe(CN)_6]$, dadurch gekennzeichnet, daß als Chromogen ein Anilinderivat der Formel I enthalten ist.

9. Anilinderivat der Formel I′

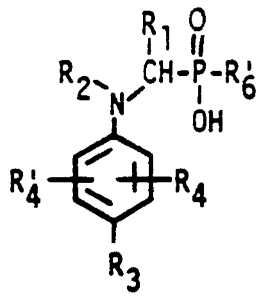

in der

$R_1$ Wasserstoff, Alkyl mit 1—6 C-Atomen, Phenyl, das zusätzlich noch durch Alkyl oder Alkoxy mit 1—6 C-Atomen oder Halogen substituiert sein kann oder Naphthyl, das gegebenenfalls teilhydriert sein kann, darstellt

$R_2$ für Wasserstoff, Alkyl mit 1—6 C-Atomen, das durch Hydroxy-, Amino-, Carboxy-, $C_1$- bis $C_6$-Alkoxycarbonyl-, $C_1$—$C_6$-Alkanoylamidogruppen, Phenyl-, und Naphthyl-($C_1$- bis $C_6$-)alkyl, wobei Phenyl noch zusätzlich durch Alkyl oder Alkoxy mit 1 bis 6 C-Atomen oder Halogen substituiert sein und Naphthyl gegebenenfalls teilhydriert sein kann und Phenyl, das zusätzlich noch durch Alkyl oder Alkoxy mit 1 bis 6 C-Atomen oder Halogen substituiert sein kann oder Naphthyl, das gegebenenfalls teilhydriert sein kann und Gruppen der Struktur

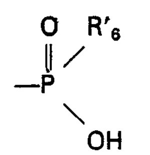

in denen $R'_6$ die unten angegebene Bedeutung hat, substituiert sein kann, steht,

$R_3$ Wasserstoff, Carboxy oder Halogen sein kann,

$R_4$ und $R_4'$ für Wasserstoff, Halogen, Carboxy-, $C_1$- bis $C_6$-Alkoxy-oder eine bevorzugt in m-Stellung stehende $C_1$- bis $C_6$-Alkylgruppe steht und

$R_1$ und $R_2$ bzw. $R_2$ und $R_4$ bzw. $R_4$ und $R_4'$ für den Fall, daß die beiden Substituenten orthoständig zueinander stehen, zusammen eine gesättigte oder ungesättigte Kohlenwasserstoffkette mit 2—6 C-Atomen bilden können, die durch Hydroxy- oder Oxogruppen substituiert sein kann,

$R'_6$ für eine Hydroxy-, eine $C_1$- bis $C_6$-Alkylgruppe oder Phenyl, das zusätzlich noch durch Alkyl oder Alkoxy mit 1 bis 6 C- Atomen oder Halogen substituiert sein kann oder Naphthyl, das gegebenenfalls teilhydriert sein kann, steht,

sowie ihre Salze mit Säuren oder Basen,

wobei wenn $R'_6$ für eine Hydroxygruppe steht, $R_3$, $R_4$ und $R'_4$ nicht gleichzeitig Wasserstoff bedeuten und $R_2$ kein Wasserstoff sein kann.

10. Verfahren zur Herstellung von Anilinderivaten der Formel I′

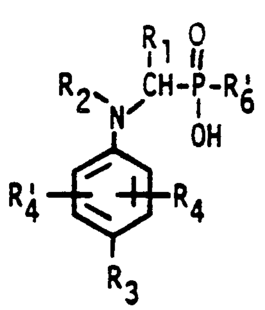

in der

$R_1$ Wasserstoff, Alkyl mit 1—6 C-Atomen, Phenyl, das zusätzlich noch durch Alkyl oder Alkoxy mit 1 bis 6C-Atomen oder Halogen substituiert sein kann oder Naphthyl, das gegebenenfalls teilhydriert sein kann, darstellt

$R_2$ für Wasserstoff, Alkyl mit 1—6 C-Atomen, das durch Hydroxy-, Amino-, Carboxy-, $C_1$- bis $C_6$-Alkoxycarbonyl-, $C_1$- bis $C_6$-Alkanoylamidogruppen, Phenyl- und Naphthyl-($C_1$- bis $C_6$-)alkyl, wobei Phenyl noch zusätzlich durch Alkyl oder Alkoxy mit 1 bis 6 C-Atomen oder Halogen substituiert sein und Naphthyl gegebenenfalls teilhydriert sein kann und Phenyl, das zusätzlich noch durch Alkyl oder Alkoxy mit 1 bis 6 C-Atomen oder Halogen substituiert sein kann oder Naphthyl, das gegebenenfalls teilhydriert sein kann und Gruppen der Struktur

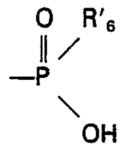

in denen $R'_6$ die unten angegebene Bedeutung hat, substituiert sein kann, steht,

$R_3$ Wasserstoff, Carboxy oder Halogen sein kann,

$R_4$ und $R_4'$ für Wasserstoff, Halogen, Carboxy-, $C_1$- bis $C_6$-Alkoxy- oder eine bevorzugt in m-Stellung stehende $C_1$- bis $C_6$-Alkylgruppe steht und

$R_1$ und $R_2$ bzw. $R_2$ und $R_4$ bzw. $R_4$ und $R_4'$ für den Fall, daß die beiden Substituenten orthoständig zueinander stehen, zusammen eine gesättigte oder ungesättigte Kohlenwasserstoffkette mit 2—6 C-Atomen bilden können, die durch Hydroxy- oder Oxogruppen substituiert sein kann,

$R'_6$ für eine Hydroxy-, eine Alkylgruppe mit 1—6 C-Atomen oder Phenyl, das zusätzlich noch durch Alkyl oder Alkoxy mit 1 bis 6 C-Atomen oder Halogen substituiert sein kann oder Naphthyl, das gegebenenfalls teilhydriert sein kann, steht

sowie ihre Salze mit Säuren oder Basen,

wobei, wenn $R'_6$ für eine Hydroxygruppe steht,

$R_3$, $R_4$ und $R'_4$ nicht gleichzeitig Wasserstoff bedeuten und $R_2$ kein Wasserstoff sein kann,

dadurch gekennzeichnet, daß man

a) ein primäres oder sekundäres Amin der Formel VI

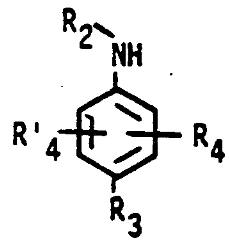

in der $R_2$, $R_3$, $R_4$ und $R'_4$ die oben angegebene Bedeutung haben und einen Aldehyd oder eine entsprechende Schiffsche Base mit einem Dialkylphosphit oder einem Phosphonigsäuredialkyester und anschließender Hydrolyse des Esters zu einer Verbindung der Formel I' umsetzt oder

b) N,O-Acetale der Struktur II

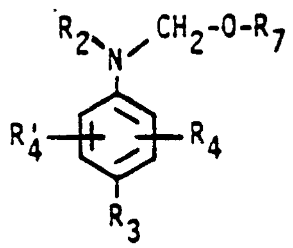

II

in der $R_2$, $R_3$, $R_4$ und $R'_4$ die obige Bedeutung haben und $R_7$ $C_1$- bis $C_6$-Alkyl darstellt oder cyclische N,O-Acetale bzw. N-Aryloxyazolidine der Formel IV

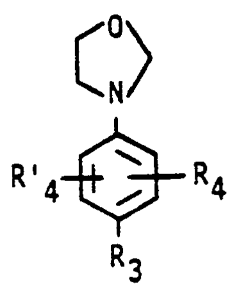

IV

in der $R_3$, $R_4$ und $R'_4$ die obige Bedeutung haben mit Trialkyphosphiten oder mit Phosphonigsäuredialkylestern der Formel III

$$R_6P(OR_5)_2 \qquad (III)$$

in der $R_5$ eine $C_1$—$C_6$-Alkylgruppe und $R_6$ $C_1$- bis $C_6$-Alkyl oder Phenyl, das zusätzlich noch durch Alkyl oder Alkoxy mit 1 bis 6 C-Atomen oder Halogen substituiert sein kann oder Napthyl, das gegebenenfalls teilhydriert sein kann bedeutet, und einem sauren Katalysator mit anschließender Hydrolyse des entstandenen Esters unter Bildung von Anilinderivaten der Formel I' mit $R_1$ = H umsetzt, oder

c) Aniline der Formel VI mit Phosphonsäureestern der Formel V

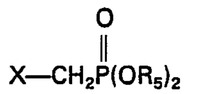

(V)

in der $R_5$ die obige Bedeutung hat und X Br, J, Tosyloxy, Benzolsulfonyloxy, Methansulfonyloxy sein kann, umsetzt und anschließend die Ester hydrolysiert oder

d) Aminoalkylphosphonsäureester der allgemeinen Formel I, in der $R_5$ $C_1$- bis $C_6$-Alkyl und $R_6$ eine $C_1$- bis $C_6$-Alkoxygruppe bedeutet, mit starken Basen und mit Alkylierungsmitteln alkyliert und anschließend die Ester hydrolysiert

und die eine erhaltenen Verbindungen ggf. mit geeigneten Säuren oder Basen in ihre Salze überführt.

**Revendications**

1. Utilisation de dérivés de l'aniline répondant à la formule I

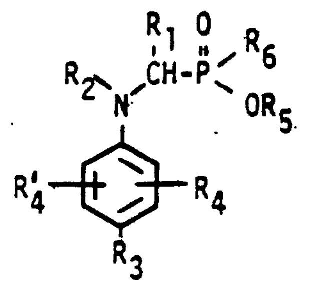

dans laquelle

$R_1$ représente l'hydrogène, un groupe alkyle comportant 1—6 atomes de C, un groupe phényle qui peut être encore substitué par un groupe alkyle ou alcoxy comportant 1—6 atomes de C ou un halogène, ou un groupe naphtyle qui peut être éventuellement partiellement hydrogéné,

$R_2$ représente l'hydrogène, un groupe alkyle comportant 1—6 atomes de C, qui peut être substitué par un groupe hydroxy, amino, carboxy, alcoxy-($C_1$ à $C_6$)-carbonyl, alcanoyl-($C_1$ à $C_6$)-amido, phényle et naphtyl-alkyle-($C_1$ à $C_6$), le groupe phényle pouvant être substitué à son tour par un groupe alkyle ou alcoxy comportant 1 à 6 atomes de C, ou un halogène, et le groupe naphtyle pouvant être éventuellement partiellement hydrogéné, et un groupe phényle, qui peut être substitué à son tour par un groupe alkyle ou alcoxy comportant 1—6 atomes de C ou un halogène, ou un groupe naphtyle qui peut être éventuellement partiellement hydrogéné, et des groupes ayant la structure

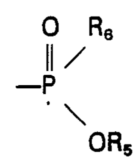

dans laquelle $R_5$ et $R_6$ ont la signification indiquée ci-après,

$R_3$ représente l'hydrogène, un groupe carboxy ou un halogène,

$R_4$ et $R_4'$ représentent l'hydrogène, un halogène, un gropue carboxy, alcoxy en $C_1$ à $C_6$ ou un groupe alkyle en $C_1$ à $C_6$, de préférence en position m, et $R_1$ et $R_2$, ou $R_2$ et $R_4$, ou $R_4$ et $R_4'$ pouvant former conjointement, dans le cas où les deux substituants sont en position ortho l'un par rapport à l'autre, une chaîne hydrocarbonée saturée ou insaturée, comportant 2—6 atomes de carbone, qui peut être substituée par des groupes hydroxy ou oxo,

$R_5$ peut représenter l'hydrogène ou un groupe alkyle comportant 1—6 atomes de C et

$R_6$ représente un groupe hydroxy, un groupe alcoxy en $C_1$ à $C_6$, un groupe alkyle en $C_1$ à $C_6$, ou un groupe phényle, qui peut être substitué à son tour par un groupe alkyle ou alcoxy comportant 1—6 atomes

de C, ou un halogène, ou un groupe naphtyle qui peut être éventuellement partiellement hydrogéné, ainsi que leurs sels formés avec des acides et des bases,

A) comme formateur de colorant conjointement avec un composant de copulation oxydable, dans la réaction de Trinder, pour la détection de

a) peroxyde d'hydrogène ou de systèmes formateurs de peroxyde d'hydrogène, ou

b) peroxydase ou de substances à action peroxydante, ou

B) conjointement avec un agent oxydant, pour la détection d'amines aromatiques ou de systèmes formateurs d'amines de ce type.

2. Utilisation selon la revendication 1, caractérisée en ce que comme composant de copulation, on utilise la 4-aminoantipyrine ou un dérivé de celle-ci ou une méthylbenzothiazolinone-hydrazone, éventuellement sulfonée.

3. Agent pour la détection de peroxyde d'hydrogène ou de systèmes formateurs de peroxyde d'hydrogène, ou de peroxydase ou de susbtances à action peroxydante, contenant un composant de copulation et un formateur de colorant pour la réaction de Trinder, et

a) un composé à action peroxydante ou

b) $H_2O_2$ ou un système produisant $H_2O_2$ ainsi que

des agents tampons et éventuellement réticulants appropriés, des activants ou d'autres additifs connus pour la réaction de Trinder, caractérisé en ce que comme formateur de colorant, il contient un dérivé de l'aniline répondant à la formule I.

4. Agent selon la revendication 3, caractérisé en ce que comme composant de copulation, on utilise la 4-aminoantipyrine ou un dérivé de cell-ci ou une méthylbenzothiazolinone-hydrazone, éventuellement sulfonée.

5. Agent pour la détection d'amines aromatiques ou de systèmes libérant celles-ci, contenant un agent oxydant ainsi que des agents tampons et éventuellement réticulants appropriés, des activateurs ou d'autres additifs connus pour la réaction de Trinder, caractérisé en ce que comme formateur de colorant, il contient un dérivé de l'aniline répondant à la formule I.

6. Agent selon la revendication 5, caractérisé en ce que comme agent oxydant, on utilise le $[Fe(CN)_6]^{3-}$, $H_2O_2$/POD, un periodate ou un persulfate.

7. Agent selon la revendication 5, pour la détection d'une amidase, caractérisé en ce que l'agent contient, comme substrat libérant une amine, un composé de formule VII

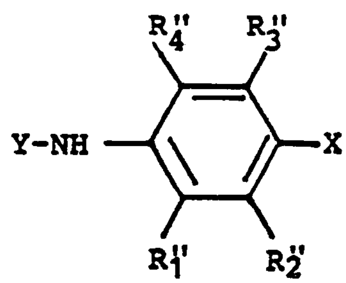

VII

dans laquelle $R''_1$—$R''_4$ représentent l'hydrogène, un halogène, un groupe alkyle comportant 1—6 atomes de C, un groupe alcoxy comportant 1—6 atomes de C, un groupe carboxy ou un groupe sulfonyle, ou bien $R''_1$ et $R''_2$ ou $R''_3$ et $R''_4$ peuvent également former un groupe alkyle ou alkylène, X' représente un groupe hydroxy ou un groupe amino, qui peut être substitué une ou deux fois par un groupe alkyle, et Y représente un acide aminé éventuellement protégé ou un groupe peptide.

8. Agent pour la détermination du temps de coagulation en une phase selon Quick, constitué de Tos-Gly-Pro-Arg-p-phénylènediamine, d'un tampon, de $K_3[Fe(CN)_6]$, caractérisé en ce que comme chromogène, il contient un dérive de l'aniline répondant à la formule I.

9. Dérivé de l'aniline répondant à la formule I'

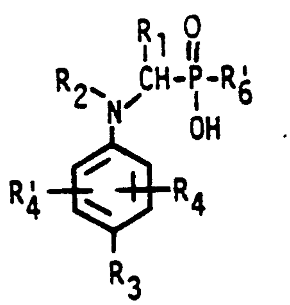

dans laquelle

$R_1$ représente l'hydrogène, un groupe alkyle comportant 1—6 atomes de C, un groupe phényle qui peut

être encore substitué par un groupe alkyle ou alcoxy comportant 1—6 atomes de C ou un halogène, ou un groupe naphtyle qui peut être éventuellement partiellement hydrogéné,

$R_2$ représente l'hydrogène, un groupe alkyle comportant 1—6 atomes de C, qui peut être substitué par un groupe hydroxy, amino, carboxy, alcoxy-($C_1$ à $C_6$)-carbonyle, alcanoyl-($C_1$ à $C_6$)-amido, phényle et naphtyl-alkyle-($C_1$ à $C_6$), le groupe phényle pouvant être encore substitué par un groupe alkyle ou alcoxy comportant 1 à 6 atomes de C, ou un halogène, et le groupe naphtyle pouvant être éventuellement partiellement hydrogéné, et un groupe phényle, qui peut être substitué par un groupe alkyle ou alcoxy comportant 1—6 atomes de C ou un halogène, ou un groupe naphtyle qui peut être éventuellement partiellement hydrogéné, et des groupes ayant la structure

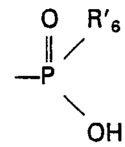

dans laquelle

$R'_6$ a la signification indiquée ci-après,

$R_3$ représente l'hydrogène, un groupe carboxy ou un halogène,

$R_4$ et $R_4'$ représentent l'hydrogène, un halogène, un groupe carboxy, alcoxy en $C_1$ à $C_6$ ou un groupe alkyle en $C_1$ à $C_6$, de préférence en position m, et $R_1$ et $R_2$, ou $R_2$ et $R_4$, ou $R_4$ et $R_4'$ pouvant former conjointement, dans le cas où les deux substituants sont en position ortho l'un par rapport à l'autre, une chaîne hydrocarbonée saturée ou insaturée, comportant 2—6 atomes de carbone, qui peut être substituée par des groupes hydroxy ou oxo,

$R'_6$ représente un groupe hydroxy, un groupe alkyle en $C_1$ à $C_6$, ou un groupe phényle, qui peut être encore substitué par un groupe alkyle ou alcoxy comportant 1—6 atomes de C, ou un halogène, ou un groupe naphtyle qui peut être éventuellement partiellement hydrogéné,

ainsi que leurs sels formés avec des acides et des bases,

étant entendu que lorsque $R'_6$ représente un groupe hydroxy, $R_3$, $R_4$ et $R'_4$ ne peuvent pas représenter simultanément l'hydrogène, et $R_2$ ne peut pas représenter l'hydrogène.

10. Procédé pour la préparation de dérivés de l'aniline répondant à la formule I'

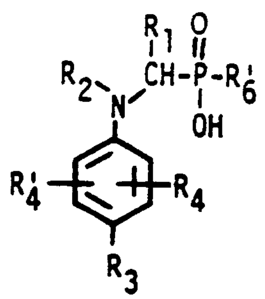

dans laquelle

$R_1$ représente l'hydrogène, un groupe alkyle comportant 1—6 atomes de C, un groupe phényle qui peut être substitué à son tour par un groupe alkyle ou alcoxy comportant 1—6 atomes de C ou un halogène, ou un groupe naphtyle qui peut être éventuellement partiellement hydrogéné,

$R_2$ représente l'hydrogène, un groupe alkyle comportant 1—6 atomes de C, qui peut être substitué par un groupe hydroxy, amino, carboxy, alcoxy-($C_1$ à $C_6$)-carbonyle, alcanoyl-($C_1$ à $C_6$)-amido, phényle et naphtyl-alkyle-($C_1$ à $C_6$), le groupe phényle pouvant être encore substitué par un groupe alkyle ou alcoxy comportant 1 à 6 atomes de C, ou un halogène, et le groupe naphtyle pouvant être éventuellement partiellement hydrogéné, et un groupe phényle, qui peut être encore substitué par un groupe alkyle ou alcoxy comportant 1—6 atomes de C ou un halogène, ou un groupe naphtyle qui peut être éventuellement partiellement hydrogéné, et des groupes ayant la structure

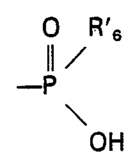

dans laquelle

$R'_6$ a la signification indiquée ci-après,

$R_3$ représente l'hydrogène, un groupe carboxy ou un halogène,

$R_4$ et $R_4'$ représentent l'hydrogène, un halogène, un groupe carboxy, alcoxy en $C_1$ à $C_6$ ou un groupe

alkyle en $C_1$ à $C_6$, de préférence en position m, et $R_1$ et $R_2$, ou $R_2$ et $R_4$, ou $R_4$ et $R_4'$ pouvant former conjointement, dans le cas où les deux substituants sont en position ortho l'un par rapport à l'autre, une chaîne hydrocarbonée saturée ou insaturée, comportant 2—6 atomes de carbone, qui peut être substituée par des groupes hydroxy ou oxo,

$R'_6$ représente un groupe hydroxy, un groupe alkyle en $C_1$ à $C_6$, ou un groupe phényle, qui peut encore substitué par un groupe alkyle ou alcoxy comportant 1—6 atomes de C, ou un halogène, ou un groupe naphtyle qui peut être éventuellement partiellement hydrogéné,
ainsi que leurs sels formés avec des acides et des bases,
étant entendu que lorsque $R'_6$ représente un groupe hydroxy, $R_3$, $R_4$ et $R'_4$ ne peuvent pas représenter simultanément l'hydrogène, et $R_2$ ne peut pas représenter l'hydrogène,
caractérisé en ce que l'on fait réagir.

a) une amine primaire ou secondaire de formule VI

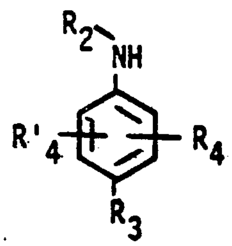

dans laquelle $R_2$, $R_3$, $R_4$ et $R'_4$ ont les significations indiquées ci-dessus, avec un aldéhyde ou une base de Schiff correspondante, avec un dialkylphosphite ou un dialkylester de l'acide phosphorique, et ensuite, on hydrolyse l'ester en un composé de formule I', ou

b) un N,O-acétal de structure II

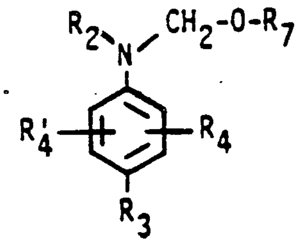

II

dans laquelle $R_2$, $R_3$, $R_4$ et $R'_4$ ont les significations indiquées ci-dessus et $R_7$ représente un groupe alkyle en $C_1$ à $C_6$, ou un N,O-acétal cyclique ou une N-aryloxazolidine de formule IV

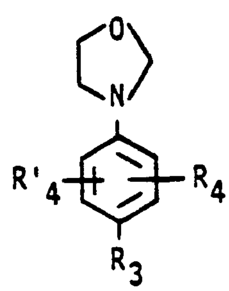

IV

dans laquelle $R_3$, $R_4$ et $R'_4$ ont les significations indiquées ci-dessus, avec des trialkylphosphites ou avec des dialkylesters de l'acide phosphonique de formule III

$$R_6P(OR_5)_2 \quad \text{(III)}$$

dans laquelle $R_5$ représente un groupe alkyle en $C_1$ à $C_6$, et $R_6$ représente un groupe alkyle en $C_1$ à $C_6$ ou un groupe phényle, qui peut être encore substitué par un groupe alkyle ou alcoxy comportant 1 à 6 atomes de C, ou par un groupe halogène, ou un groupe naphtyle qui peut être éventuellement partiellement hydrogéné,
et un catalyseur acide, puis on hydrolyse l'ester obtenu en formant le dérivé de l'aniline de formule I', avec $R_1$ = H, ou

c) une aniline de formule VI avec des esters de l'acide phosphonique de formule V

$$X-CH_2\overset{\overset{\displaystyle O}{\|}}{P}(OR_5)_2 \qquad (V)$$

dans laquelle $R_5$ a la signification mentionnée ci-dessus, et

X peut représenter Br, I, un groupe tosyloxy, benzènesulfonyloxy, méthanesulfonyloxy, et ensuite, on hydrolyse l'ester, ou

d) un ester d'acide aminoalkylphosphonique de formule (I), dans laquelle $R_5$ représente un groupe alkyle en $C_1$ à $C_6$ représente un groupe alcoxy en $C_1$ à $C_6$, avec une base forte et on alkyle à l'aide d'un agent d'alkylation et ensuite, on hydrolyse l'ester,

et on transforme éventuellement le composé obtenu en un de ses sels, à l'aide d'un acide ou d'une base appropriée.

**Claims**

1. Use of aniline derivatives of the general formula:

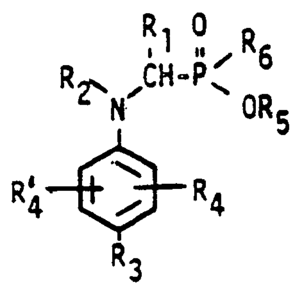

(I)

in which

$R_1$ represents hydrogen, alkyl with 1—6 C-atoms, phenyl, which can be additionally substituted by alkyl or alkoxy with 1—6 C-atoms or halogen, or naphthyl, which can possibly be partly hydrogenated,

$R_2$ stands for hydrogen, alkyl with 1—6 C-atoms, which can possibly be substituted by hydroxyl, amino, carboxyl, $C_1$- to $C_6$-alkoxycarbonyl, $C_1$- to $C_6$-alkanoylamido groups, phenyl and naphthyl-($C_1$ to $C_6$-) alkyl, whereby phenyl can also be additionally substituted by alkyl or alkoxy with 1 to 6 C-atoms or halogen and naphthyl can possibly be partly hydrogenated, and phenyl, which can also be additionally substituted by alkyl or alkoxy with 1 to 6 C-atoms or halogen, or naphthyl, which can possibly be partly hydrogenated, and groups of the structure:

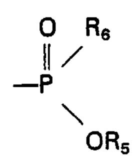

in which $R_5$ and $R_6$ have the meaning given below,

$R_3$ can be hydrogen, carboxyl or halogen,

$R_4$ and $R'_4$ stand for hydrogen, halogen, carboxyl, $C_1$- to $C_6$-alkoxy or a $C_1$- to $C_6$-alkyl group preferably standing in the *m*-position and

$R_1$ and $R_2$ or $R_2$ and $R_4$ or $R_4$ and $R'_4$, for the case that the two substituents stand ortho to one another, can together form a saturated or unsaturated hydrocarbon chain with 2—6 C-atoms, which can be substituted by hydroxyl or oxo groups,

$R_5$ can be hydrogen or an alkyl group with 1—6 C-atoms and

$R_6$ stands for a hydroxyl, a $C_1$- to $C_6$-alkoxy, a $C_1$- to $C_6$-alkyl or phenyl, which can additionally be substituted by alkyl or alkoxy with 1—6 C-atoms or halogen, or naphthyl which can possibly be partly hydrogenated, as well as their salts with acids or bases,

A) as colour former, together with an oxidisable coupling component, in the Trinder reaction for the detection of

a) hydrogen peroxide or hydrogen peroxide-forming systems or

b) peroxidase or peroxidate-acting substances or

B) together with an oxidation agent for the detection of aromatic amines or of systems forming such amines.

2. Use according to claim 1, characterised in that, as coupling component, 4-aminoantipyrine or a derivative thereof or an optionally sulphonated methylbenzthiazolinone hydrazone is used.

3. Agent for the detection of hydrogen peroxide or of hydrogen peroxide-forming systems or of peroxidase or of peroxidase-acting systems containing a coupling component and a colour former for the Trinder reaction and

a) a peroxidate-active compound or

b) $H_2O_2$ or an $H_2O_2$-producing system,

as well as suitable buffers and optionally wetting agents, activators or other adjuvants known for the Trinder reaction, characterised in that, as colour former, it contains an aniline derivative of formula I.

4. Agent according to claim 3, characterised in that, as coupling component, 4-aminoantipyrine or a derivative thereof or an optionally sulphonated methylbenzthiazolinone hydrazone is used.

5. Agent for the detection of aromatic amines or of systems liberating these, containing an oxidation agent, as well as suitable buffers and optionally wetting agents, activators or other adjuvants known for the Trinder reaction, characterised in that, as colour former, it contains an aniline derivative of formula I.

6. Agent according to claim 5, characterised in that $[Fe(CN)_6]^{3-}$, $H_2O_2$/POD, periodate or persulphate is used as oxidation agent.

7. Agent according to claim 5 for the detection of an amidase, characterised in that, as substrate liberating amine, the agent contains a compound of the formula VII

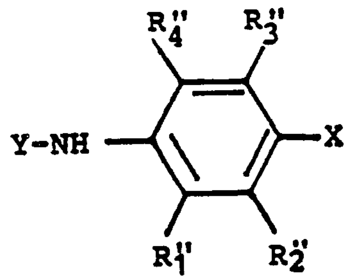

VII

in which $R''_1$—$R''_4$ represent hydrogen, halogen, alkyl with 1—6 C-atoms, alkoxy with 1—6 C-atoms, a carboxyl or sulphonyl group or $R''_1$ and $R''_2$ or $R''_3$ and $R''_4$ can also form an alkyl or alkylene bridge, X represents a hydroxyl group or an amino group which can be substituted once or twice by alkyl and Y an optionally protected amino acid or peptide group.

8. Agent for the determination of the one-stage coagulation time according to Quick, consisting of Tos-Gly-Pro-Arg-*p*-phenylenediamine, buffer, $K_3[Fe(CN)_6]$, characterised in that, as chromogen, it contains an aniline derivative of formula I.

9. Aniline derivative of the formula I′

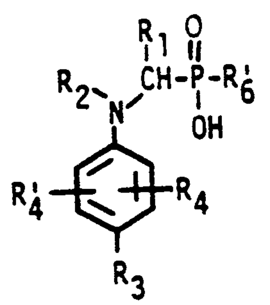

(I′)

in which $R_1$ represents hydrogen, alkyl with 1—6 C-atoms, phenyl, which can additionally be substituted by alkyl or alkoxy with 1—6 C-atoms or halogen, or naphthyl which can optionally be partly hydrogenated, $R_2$ stands for hydrogen, alkyl with 1—6 C-atoms, which can be substituted by hydroxyl, amino, carboxyl, $C_1$ to $C_6$-alkoxycarbonyl, $C_1$—$C_6$-alkanoylamido groups, phenyl and naphthyl-($C_1$ to $C_6$)-alkyl, whereby phenyl can additionally be substituted by alkyl or alkoxy with 1 to 6 C-atoms or halogen and naphthyl can possibly be partly hydrogenated, and phenyl, which can additionally be substituted by alkyl or alkoxy with 1 to 6 C-atoms or halogen, or naphthyl, which can possibly be partly hydrogenated, and groups of the structure:

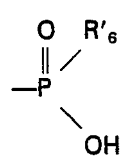

in which $R'_6$ has the meaning given below, $R_3$ can be hydrogen, carboxyl or halogen, $R_4$ and $R'_4$ stand for hydrogen, halogen, carboxyl, $C_1$—$C_6$-alkoxy or a $C_1$ to $C_6$-alkyl group preferably standing in the *m*-position

and $R_1$ and $R_2$ or $R_2$ and $R_4$ or $R_4$ and $R'_4$, for the case that the two substituents stand ortho to one another, can together form a saturated or unsaturated hydrocarbon chain with 2—6 C-atoms, which can be substituted by hydroxyl or oxo groups, $R'_6$ stands for a hydroxyl, a $C_1$ to $C_6$ alkyl group or phenyl, which can additionally also be substituted by alkyl or alkoxy with 1 to 6 C-atoms or halogen, or naphthyl, which can possibly be partly hydrogenated, as well as their salts with acids or bases, whereby, when $R'_6$ stands for a hydroxyl group, $R_3$, $R_4$ and $R'_4$ do not simultaneously signify hydrogen and $R_2$ cannot be hydrogen.

10. Process for the preparation of aniline derivatives of the formula I'

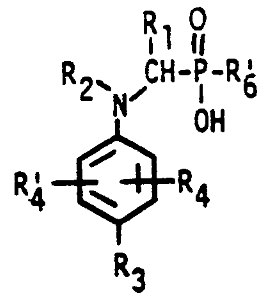

in which $R_1$ represents hydrogen, alkyl with 1 to 6 C-atoms, phenyl, which can be additionally substituted by alkyl or alkoxy with 1 to 6 C-atoms or halogen, or naphthyl, which can possibly be partly hydrogenated, $R_2$ stands for hydrogen, alkyl with 1—6 C-atoms, which can be substituted by hydroxyl, amino, carboxyl, $C_1$—$C_6$-alkoxycarbonyl, $C_1$—$C_6$-alkanoylamido groups, phenyl and naphthyl ($C_1$ to $C_6$)-alkyl, whereby phenyl can also be additionally substituted by alkyl or alkoxy with 1 to 6 C-atoms or halogen and naphthyl can possibly be partly hydrogenated, and phenyl, which can additionally also be substituted by alkyl or alkoxy with 1 to 6 C-atoms or halogen, or naphthyl, which can possibly be partly hydrogenated, and groups of the structure:

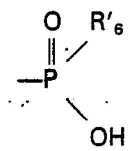

in which $R'_6$ has the meaning given below, $R_3$ can be hydrogen, carboxyl or halogen, $R_4$ and $R'_4$ hydrogen, halogen, carboxyl, $C_1$ to $C_6$ alkoxy or a $C_1$—$C_6$ alkyl group preferably standing in the *m*-position, $R_1$ and $R_2$ or $R_2$ and $R_4$ or $R_4$ and $R'_4$, for the case that the two substituents stand ortho to one another, can together form a saturated or unsaturated hydrocarbon chain with 2—6 C-atoms which can be substituted by hydroxyl or oxo groups, $R'_6$ stands for a hydroxyl, an alkyl group with 1—6 C-atoms, or phenyl, which can additionally also be substituted by alkyl or alkoxy with 1—6 C-atoms or halogen, or naphthyl which can possibly be partly hydrogenated, as well as their salts with acids or bases, whereby when $R'_6$ stands for a hydroxyl group, $R_3$, $R_4$ and $R'_4$ cannot simultaneously be hydrogen and $R_2$ cannot be hydrogen, characterised in that one

a) reacts a primary or secondary amine of the formula VI

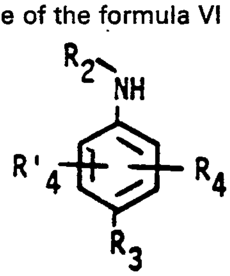

in which $R_2$, $R_3$, $R_4$ and $R'_4$ have the above-given meanings, and an aldehyde or a corresponding Schiff base with a dialkyl phosphite or a phosphonous acid dialkyl ester and subsequent hydrolysis of the ester to a compound of the formula I', or

b) reacts N,O-acetals of the structure II

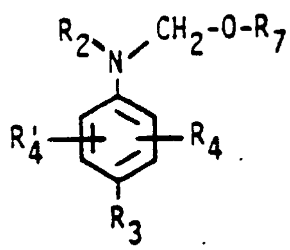

in which $R_2$, $R_3$, $R_4$ and $R'_4$ have the above meaning and $R_7$ represents $C_1$ to $C_6$-alkyl, cyclic N,O-acetals or N-aryloxazolidines of the formula IV

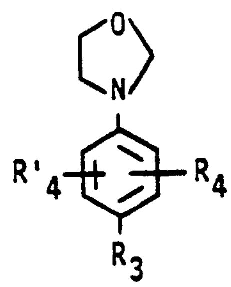

in which $R_3$, $R_4$ and $R'_4$ have the above meaning, with trialkyl phosphites or with phosphonous acid dialkyl esters of the formula III

$$R_6P(OR_5)_2$$

in which $R_5$ signifies a $C_1$—$C_6$-alkyl group and $R_6$ $C_1$ to $C_6$-alkyl or phenyl, which can additionally also be substituted by alkyl or alkoxy with 1 to 6 C-atoms or halogen, or naphthyl, which can possibly be partly hydrogenated, and an acidic catalyst with subsequent hydrolysis of the resultant ester with formation of aniline derivatives of the formula I' with $R_1 \neq H$, or

c) reacts anilines of the formula VI with phosphonic acid esters of the formula V

$$X\text{—}CH_2\overset{O}{\overset{\|}{P}}(OR_5)_2$$

in which $R_5$ has the above meaning and X can be Br, I, tosyloxy, benzenesulphonyloxy, methanesulphonyloxy, and subsequent hydrolysis of the esters, or

d) alkylates aminoalkylphosphonic acid esters of general formula I, in which $R_5$ signifies $C_1$ to $C_6$-alkyl and $R_6$ a $C_1$ to $C_6$-alkoxy group, with strong bases and with alkylation agents and subsequently hydrolyses the esters, and converts the compounds obtained possibly with suitable acids or bases into their salts.